# EUROPEAN PATENT SPECIFICATION

(11) **EP 0 820 225 B1**
(45) Date of publication and mention of the grant of the patent: **26.03.2003**
(21) Application number: 96910628.5
(22) Date of filing: 28.03.1996
(51) Int. Cl.: A01N 25/02, A01N 37/02, A01N 37/06

(54) **FAST ACTING AND PERSISTENT TOPICAL ANTISEPTIC**
SCHNELL UND ANHALTEND WIRKENDES TOPISCHES ANTISPETIKUM
ANTISEPTIQUE A USAGE LOCAL, A ACTION RAPIDE ET A EFFET REMANENT

(30) Priority: 31.03.1995 US 412327
(43) Date of publication of application: 28.01.1998
(73) Proprietor: Guthery, B., Eugene, Broken Bow, OK 74728 (US)
(72) Inventor: Guthery, B., Eugene, Broken Bow, OK 74728 (US)
(74) Representative: UEXKÜLL & STOLBERG
(86) International application number: PCT/US96/04258
(87) International publication number: WO 96/029867

(56) References cited:
- EP-A- 0 099 209
- EP-A- 0 375 827
- CH-A- 329 198
- DE-C- 731 718
- GB-A- 1 126 953
- GB-A- 1 164 981
- GB-A- 2 001 649
- US-A- 4 137 311
- US-A- 4 161 526
- US-A- 5 360 788
- SEYMOUR S. BLOCK: "Disinfection, Sterilization, and Preservation" 1991 , 4TH. ED.; LEA & FEBIGER , PHILADELPHIA, US XP002015577 pages 67-68, 199-200 and 642-654 see in particular pages 199-200 and page 647, last paragraph - page 648, second paragraph
- PATENT ABSTRACTS OF JAPAN vol. 13, no. 423 (C-638) [3771] , 20 September 1989 & JP,A,01 160922 (LION CORP.), 23 June 1989,

## Description

### TECHNICAL FIELD

This invention relates to topical antiseptic compositions.

### BACKGROUND OF THE INVENTION

Humans have recognized the need for antiseptics since ancient times. An antiseptic is an agent used on or in living tissue to inhibit the growth and activity of microorganisms or to destroy them. Even though the early humans did not understand the reasons why infection occurred, they did understand that certain compositions reduced the likelihood of infection and, therefore, increased the likelihood that a patient to whom the composition was applied was going to survive an injury or infection. The compositions that were most effective were naturally occurring antiseptics. In the late 1800's, scientists such as Lister began to recognize the cause of infection and that treatment of a wound with an antiseptic would significantly improve the chance of survival of the patient. Antiseptics also made possible the removal of microorganisms from surgeon's skin prior to operations which could have otherwise infected the patient, potentially leading to death.

Currently, there are several conventional antiseptics which are effective against microorganisms which cause mammalian infection, approved for use by the Food and Drug Administration, and commonly used in hospitals. These conventional antiseptics provide varying degrees of antimicrobial activity. Antimicrobial activity is classified as high, i.e., killing vegetative bacteria, acid-fast bacteria, bacterial spores, fungi, lipid and medium-sized viruses, and nonlipid and small viruses; intermediate, i.e., killing all the above except for possibly bacterial spores and nonlipid and small viruses; and low, i.e., killing vegetative bacteria, lipid and medium-sized viruses and possibly fungi but ineffective against acid-fast bacteria, bacterial spores, and nonlipid and small viruses. Alcohols such as ethanol and isopropanol at 70% (v/v) provide intermediate antimicrobial action. Iodophor in concentrations of 1-2 milligram per liter with 1%-2% available iodine are considered intermediate to low antimicrobial antiseptics. Chlorhexidine (0.75%-4.0%), hexachlorophene (1%-3%), parachlorometaxylenol (0.5%-4%), and mercurial compounds (0.1%-0.2%) are antiseptics of low antimicrobial activity.

The degree of antimicrobial action for a given antiseptic is influenced by several factors. As indicated by the classification given above, the type of microorganism greatly affects microbicidal level, with spore-formers and certain viruses such as nonlipid and small viruses being the most difficult to kill. Under a given set of circumstances, the higher the level of microbial contamination, the longer the exposure to the antiseptic is required for microbicidal activity. The ability of the antiseptic to penetrate to all contaminated surfaces also affects the degree of killing.

While exposure of microorganisms maintained on a smooth surface or in a liquid solution to many conventional antiseptics (such as alcohols) will eventually kill virtually all of the microorganisms, the skin presents some unusual problems. Even though conventional antiseptics are effective at killing a vast majority of the microorganisms present on the surface, there is a continuing problem with a small percentage of the microorganisms remaining viable in the superficial and deeper layers of the skin. For example, if skin originally contains one million bacteria per square centimeter and there is a four log reduction in the number of bacteria subsequent to application of the antiseptic, there remains approximately one hundred bacteria still viable in the superficial and deeper layers of the skin. Skin bacteria have been described as "transients" and "residents". The transient bacteria lie free on the skin or are loosely attached. The transient bacteria are removed rather easily. The resident bacteria are very difficult, if not impossible, to remove. These bacteria may be partially hidden from antiseptics; in particular, the skin has numerous sweat ducts and hair follicles on a microscopic level that allows microorganisms to be beneath the exposed surface and potentially miss being killed by antiseptic treatments. But more importantly, the resident bacteria are within the stratum corneum and are not killed with conventional antiseptics.

The quality of persistence, sometimes also referred to as residual activity or substantivity, refers to the ability of an antiseptic to continue to kill once it is applied. According to the U.S. Food and Drug Administration (FDA), "a property [of an antiseptic] such as persistence, which acts to prevent the growth or establishment of transient mircoorganisms as part of the normal baseline or resident flora, would be an added benefit." *Federal Register* 59:31407 (June 17,1994). Most conventional antiseptics have a relatively short persistence, such that within a few hours the quantity of microorganisms on tissue will return to the original numbers. For example, if a conventional alcohol antiseptic is utilized on the skin, the number of microorganisms per square centimeter are effectively back to their original level after approximately three hours. Furthermore, during that three hour period the bacterial colonies are expanding significantly with each passing hour, that is, bacteria are multiplying during that time and do not just rebound to normal levels at the end of the three hour time period.

Chlorhexidine gluconate, reported to be the best persistent antiseptic available, has demonstrated antiseptic activity for about six hours. Larson, E., "APIC guidelines for infection control practice," *American J Infection Control* 16:253-66 (1988).

As it is desirable to maintain medical procedures as free of microorganisms as possible, it is desirable to have persistence associated with an antiseptic that both maintains the microorganism count at as low a level as possible and for as long as possible. This is especially true where there is an open wound, or where a patient is being operated upon for many hours and it is important to minimize the microorganism count of both the operative site and the hands of the surgeon for as long as possible. Moreover, this is also true around a chronic indwelling device. In such cases, if the microorganism level returns to greater than fifteen colony forming units per square centimeter on the skin of the patient, the likelihood is that the site of the wound, procedure, device, or operation will also become infected.

While certain conventional antiseptics are highly effective against microorganisms causing mammalian infection, they do not provide in a single composition a very broad spectrum, quick-acting and persistent antiseptic that kills as many different microorganisms as possible, especially those that are most likely to cause serious injury to humans due to infection. Currently available antiseptics still often leave a variety of microorganisms in the treated tissue which may lead to infection with a significant increase in morbidity and mortality. Consequently, there has been a continuing need to improve antiseptics which will kill as many microorganisms as possible on the surface and in the epidermis of the skin in a short period of time and, especially, produce persistence to maintain such kill for an extended period of time.

Novel antiseptics have now been found which are very broad spectrum having antimicrobial activity against microorganisms such as but not limited to vegetative bacteria, yeasts, molds, and viruses, quick-acting and persistent antiseptics, which are non-irritating, have reasonably pleasant characteristics associated with the senses of the persons using the antiseptic such as having a pleasant smell, and have a good tactile feel when applied to the skin. These antiseptics are easily modified to be used over a broad range of uses such as a topical antiseptic applied to the skin of humans or other mammals; a skin preparation antiseptic for use prior to medical operations; a hand wash for doctors and other medical practitioners; an antiseptic at medical appliance invasive sites such as locations where needles or tubes are placed within the skin or open wounds; a microorganism barrier or an antiseptic within various orifices such as the ear or vagina; an antiseptic to be utilized on sensitive membranes such as mucous membranes, eyes or genitalia; an antiseptic to be used in the treatment of inflammatory dermatoses, e.g., acne, athlete's foot, psoriasis and fungal infections; an antiseptic treatment for use on animals to prevent infection, e.g., as a treatment against bovine mastitis; and an antiseptic to be applied to the surface of devices which are subsequently applied to skin such as gloves, drapes, or tape.
JP,A,01-160922 discloses a germicide composition having prolonged germicidal effect and containing a polyacrylic acid long-chain alkyl or alkenyl amine salt, a pyrithione based compound and an alcohol. US,A,4137311 teaches a suspension of hydrocortizone and a pyrithione salt in ethanol as well as formulations containing a pyrithione salt, a fatty acid or a fatty acid ester.
A combination of fast-acting alcohols with other microbicides like the persistent nitrogen compounds chlorhexidine or iodophors, emollients and refatting agents is recommended by Seymour S. Block: "Disinfection, Sterilization, and Preservation" 1991, 4th Ed, Lea & Febiger, Philadelphia, US, pages 67-68, 199-200 and 642-654.
EP,A,0099209 describes aqueous disinfectant solutions containing an alcohol and a combination of a biguanide and a quaternary ammonium salt.
EP,A,0375827 describes a disinfectant composition comprising an emollient like, for example, diphtalates or di-adipates, a disinfectant like, for example, chlorhexidine or quaternary ammonium salt, and ethanol.
None of the prior art documents discloses, however, the antiseptic formulation of the invention.

### SUMMARY OF THE INVENTION

According to the invention, new antiseptic formulations for topical application are provided as stable, non-irritating solutions or emulsions effective for quickly killing microorganisms present in a treated area and for providing a continued killing or inhibitory effect for greater than six hours. These formulations each contain at least one antimicrobial alcohol selected from the group consisting of ethanol, isopropanol, n-propanol and mixtures thereof in combination with at least one persistent agent effective for prolonging the antimicrobial activity of said antimicrobial alcohol.

This persistent agent is a bispyrithione compound, in particular zinc pyrithione, magnesium pyrithione or mixtures thereof. Most preferred is a bispyrithione compound at from about 0,0005% to about 10% (w/v). To this combination is added in one embodiment of the invention, a fatty acid, preferably having a chain length of from 2 to 20 carbons, and most preferable, linoleic or linolenic acid, por a fatty acid ester, preferably having a chain length of from 2 to 24 carbons, and most preferable, glycerol monolaurate. In which case the bis pyrithione is zinc pyrithione and the formulation has an acidic pH.

In another embodiment an emulsifying agent selected from the group consisting of ethoxylated cetyl alcohol, ethoxylated stearyl alcohol and mixtures thereof, and emulsifying waxes essentially comprising cetyl and stearyl alcohols is added.

The antiseptic formulation of the present invention may also contain a penetration controlling agent such as unsaturated long-chain fatty acids, esters of fatty acids, propylene glycol, medium-chain saturated fatty acids, medium-chain alcohols (C8 through C14), amine oxides, mineral oil, petrolatum, cetyl alcohol, stearyl alcohol, polymers of linoleic acid, and mixtures thereof.

Optionally, the antiseptic formulations may contain a preservative; an emulsifying agent; 2-deoxy-D-glucose; hyaluronic acid; glycyrrhetinic acid; or an iodophor. A preferred embodiment comprises from about 30% to about 98% (v/v) alcohol, from about 0.0001% to about 15% (w/v) bispyrithione compound, and from about 1% to about 69% (v/v) water. A more preferred embodiment comprises from about 40% to about 95% (v/v) alcohol, from about 0.0001% to about 12% (w/v) bispyrithione compound, and from about 26% to about 60% (v/v) water. A most preferred embodiment comprises about 70% (v/v) alcohol, from about 0.0001% to about 10% (w/v) bispyrithione compound, and from about 7.5% to about 30% (v/v) water. The preferred pH range is between 2 and 5; more preferred, between 3 and 4, and most preferred, less than 7.

### DETAILED DESCRIPTION

The present invention is directed to a topical antiseptic that advantageously provides both a very fast acting antimicrobial action, or quick kill, of existing microorganisms on the surface of living tissue and is persistent in preventing the return of microorganisms to the treated surface. Fast acting antimicrobials provide profound, immediate bactericidal properties indicated by a significant log drop in the number of organisms obtained by culturing the application site within a few minutes post-application when compared to microbial counts taken prior to application of the antimicrobial.

The property of persistence is provided by a composition which is bound by physiochemical forces to cause the antimicrobial composition to establish a reservoir in and/or on the stratum corneum. This reservoir exerts an antimicrobial effect for several hours or days beyond the last application of the antimicrobial composition, because the antimicrobial composition is retained in the stratum corneum until the cells to which the antimicrobial composition is fixed are shed in the normal process of desquamation. The degree of persistence is measured by the length of time required for microflora to be fully restored to baseline counts following use or repeated use of the antimicrobial composition.

In addition to the quick kill components and persistent components of the present invention, the antiseptic compositions may also contain penetration enhancing components, chelating agent components, antioxidants, emulsifiers, colorings, texturings, and over-the counter (OTC) treatments.

It is preferred to have an antiseptic that not only quickly kills microorganisms with a high log ratio kill on contact with the skin, but also has the ability to: 1) penetrate throughout the levels of the skin where microorganisms reside without irritating or doing substantial harm to the treated area, 2) bind to lipids or proteins in the intercellular layers of the epidermis to produce a prolonged kill or persistence, and 3) bind to lipids or proteins on the surface of the skin so as to have a prolonged or persistent kill where organisms are killed as they attempt to recolonize the skin surface from their resident sources. Persistence in antimicrobials is especially important in adolescents and adults. In neo-nates where the skin is only a few cell layers thick, with less resident flora, persistence can be achieved with less penetrating ability.

Antiseptics of the present invention are useful over a wide range of antiseptic uses and may be easily modified for efficacy requiring specific characteristics. For example, these antiseptics can be utilized as an antiseptic skin preparation for a patient undergoing an operation; a pre-operative scrub for the hands of physicians or other medical technicians; a health care personnel hand wash; a routine hand wash; an antiseptic around invasive sites for medical appliances such as needles and catheters; a treatment for inflammatory dermatoses such as acne and topical fungal infections; an antiseptic for use in sensitive areas such as mucous membranes, eyes, ears, genitalia, and vagina; and as an antiseptic applicable to ways in which other conventional antiseptics and antimicrobials are utilized.

In particular, the antiseptic compositions of the present invention comprise a quick-kill component(s) and a persistent component(s). It is especially desirable that these two components along with other ingredients of the invention render the tissue treated by the antiseptic hydrophobic and lipophilic. The persistent component(s) binds to either the skin surface and/or intercellular structures within the epidermis.

The quick-kill component of the antiseptic composition of the present invention comprises an alcohol which is microbicidal, relatively inexpensive, and relatively nontoxic with topical application. Alcohols also tend to have a cleansing action and evaporate rapidly, helping to desiccate the skin and to produce an environment that is incompatible with microorganism survival.

Alcohols usable as a quick-kill component of the present invention are the aliphatic alcohols ethyl, isopropyl, and propyl alcohol. The bactericidal action of alcohols increases with the molecular weight of the alcohol except for the tertiary alcohols. The propyl alcohols, including n-propanol and isopropyl alcohol, are, in general, the highest molecular weight aliphatic alcohols that are fully miscible with water in all proportions and are commonly used as antiseptics.

N-propanol is the preferred quick-kill agent in accordance with the present invention as it is the strongest bactericide against most microorganisms of interest as well as the longest carbon chain length alcohol which is fully miscible with water in all proportions, compatible with topical application to the skin, and commonly available. N-propanol may be used by itself as a quick-kill component or in combination with other alcohols and other quick-kill agents.

In the present invention, higher chain length alcohols may also be used in mixtures. For example, amyl alcohol has very good microbicidal activity and slower evaporation. Alcohols such as n-decanol, a penetration enhancing agent, may also be useful when blended with other lower molecular weight alcohols.

While alcohols are fairly effective at killing microorganisms at neutral pH, this effectiveness increases dramatically at a lower pH. Consequently, it is desirable that compositions in which alcohols are the sole quick kill component should utilize the following pH levels: for intact skin, pH 4 or less, preferably from about 2 to about 3.5; for vagina, pH of about 4.5; for the ear canal, pH of about 4.5; for the external ear, pH of about 3.5 to about 4.5; for the eye, pH of about 7.2 to about 7.8, preferably at about 7.5; and for mucous membranes, pH of about 3.7 to about 5.5. Other ingredients added to the composition of the present invention may modify the pH and, therefore, the pH of the overall composition should be adjusted subsequent to addition of all of the components. For example, the solution may be adjusted with a phosphoric acid/monobasic sodium phosphate buffer, glacial acetic acid/sodium acetate buffer, citric acid/sodium citrate buffer, or combinations of organophosphonates such as Dequest 2010, 2060, and 2016 (Monsanto Company, St. Louis, MO).

The effectiveness of the quick-kill component is also concentration-related. In particular, most alcohols acting against common, non-spore forming bacteria under moist conditions will be ineffective against many microorganisms at concentrations less than 30% volume/volume (v/v) but fairly effective above 40% (v/v) with a time exposure of one minute. However, under either wet or dry conditions, alcohol concentrations of 60% to 70% (v/v) are most effective against microorganisms of interest. Alcohol concentrations of 100% are not useful in the present invention as some water needs to be present in the alcohol for it to be bactericidal. In the antiseptic compositions of the present invention, most alcohols preferably are utilized in concentrations ranging of from about 50% to about 70% (v/v) with the latter being the most preferred. For example, 70% (v/v) N-propanol is the most preferred quick-kill agent.

In accordance with the present invention, aromatic alcohols may be utilized advantageously in conjunction with said aliphatic alcohols such as n-propanol. The preferred aromatic alcohols include phenylethyl alcohol, benzyl alcohol, and phenoxyethyl alcohol.

Additional components may be used in conjunction with the alcohols as the quick-kill component. Many of the previously utilized or conventional antiseptics fit this criteria, including chlorhexidine gluconate, iodine, iodophors, phenol derivatives, quaternary ammonium compounds, certain heavy metals, para-chloro-meta-xylenol (PCMX), and 5-chloro-2-(2,4-dichloro-phenoxy)phenol (Triclosan).

Some of the most effective known antiseptic compositions include chlorhexidine gluconate in combination with alcohols such as isopropyl alcohol, ethyl alcohol, n-propanol or their mixtures. It is possible in accordance with the present invention to utilize chlorhexidine in conjunction with an alcohol as the quick-kill component; however, the pH of the composition must then be maintained above what is otherwise desirable for the composition. In particular, the composition containing chlorhexidine gluconate must be within the pH range of 5 to 7 in order to remain stable.

In conjunction with the quick kill component, the present invention also comprises a persistent component. The main purpose for the inclusion of a persistent component is that the substantivity or residual activity of the quick-kill components, especially the alcohols, is relatively short lived. Most of the currently used antiseptics have a residual activity of one to six hours. For example, a combination of alcohol and chlorhexidine gluconate with multiple applications has been reported to have residual microbicidal activity for up to six hours. Larson, E.L., "APIC guideline for use of Topical antimicrobial agents," *Am J Infect Control* 22:25A-47A (1994). In order to achieve persistence in excess of six hours, especially up to twenty-four hours or beyond, it is necessary to incorporate one or more of the persistent components of the present invention.

Persistent components used in addition to bispyrithiones (an below) are lipid or lipid-like materials selected from a group consisting of fatty acids and fatty acid esters. It is noted that the preferred lipid materials of the present invention for use as the persistent component are naturally occurring materials within the human. These lipid components are also antimicrobial, thus providing additional quick-kill activity in conjunction with the quick-kill component.

In particular, the lipid component is preferably a free fatty acid. The free fatty acid may be saturated or unsaturated, straight or branched with chain lengths of two to thirty carbons. As used herein short-chain length fatty acids will have between one and five carbon atoms; medium-chain fatty acids, between six and twelve carbon atoms; long-chain fatty acids, between thirteen and eighteen carbon atoms; and very long-chain fatty acids, greater than eighteen carbons atoms. Because of their special effectiveness against different ranges of microorganisms and penetrating ability, many of the compositions of the present invention will utilize more than one range of free fatty acids, or their polymers, i.e., dimers, trimers, and tetrameres.

A preferred medium-chain saturated fatty acids is C12, or lauric acid. For the long-chain fatty acids, linolenic and linoleic acids are preferred for non-inflamed skin, while linoleic acid or gamma-dihomolinolenic acid are preferred for inflamed skin.

The saturated or unsaturated free fatty acids above C16 are generally preferred over the lower chain fatty acids because they tend to be less irritating to the skin. The free fatty acids are also suitable for use on the skin as they are a natural component of the body. While purified, single component free fatty acids, e.g., 100 % pure linolenic or linoleic acids, are comparatively very expensive to obtain, saturated and unsaturated fatty acid mixtures are readily available as hydrolysates of various oils such as linseed oil, coconut oil, corn oil, soybean oil, evening primrose oil, borage oil, wheat germ oil and the like. These oils are often a very good source of linolenic and linoleic acids.

As antimicrobials, certain fatty acids are especially effective against certain types of microorganisms. Short-chain saturated free fatty acids are especially effective in killing gram-negative bacteria. Medium-chain free fatty acids are most effective against yeasts at neutral pH and aiding penetration into the stratum corneum. Long-chain unsaturated fatty acid tends to have the greatest activity against gram-positive microorganisms, with the activity typically increasing as the number of double bonds within the unsaturated fatty acids increases. The most microbicidal monosaturated fatty acid is C16:1, and the most active polyunsaturated fatty acid is C18:2. Typically, the cis form of the fatty acid is preferred over the trans. It is also noted that acetylenic fatty acids have a higher activity against fungus than ethylenic fatty acids.

The long chain unsaturated free fatty acids such as linoleic acid (C18:2) provided in commercial products such as Emery 305 or Emery 315 (Henkle Corporation, Emery Group, Cincinnati, OH) and oils supplying linoleic acid, such as safflower oil, sunflower oil, wheat germ oil, evening primrose oil, or sesame seed oil, are preferred antimicrobials. Also, linoleic acid is found to function as a false substrate for arachidonic acid metabolism which normally results in inflammation. Examples where linoleic acid could function both as an antimicrobial and an anti-inflammatory agent would include acne, psoriasis, athlete's foot, atopic dermatitis and eczema. Polymers of linoleic acid, i.e., dimer, trimer, and polybasic acids could be incorporated to function as an antimicrobial agent and reduce irritation from various detergents or irritants. Linolenic acid (C 18:3) should only be used on intact, non-inflamed skin as this compound is very pro-inflammatory if used on inflamed skin. This is because linolenic acid from the Omega 3 series can be elongated by skin enzymes to pro-inflammatory products, e.g., arachidonic acid. Gamma-dihomo-linolenic acid (C20:3) from the Omega 6 fatty acids are metabolized by the skin to anti-inflammatory prostaglandin E-1. So the selection of the long chain unsaturated fatty acid depends on whether one is treating inflamed or non-inflamed skin.

Fatty acid esters of the present invention may be:
1) monoglycerol esters of fatty acids with carbon chain lengths from two to twenty-four; 2) methyl esters of fatty acids including alpha-hydroxymethyl esters; 3) poly (tri-, hexa- and deca-) glycerol esters with carbon chain lengths from two to twenty-four, saturated or unsaturated, straight or branched; 4) sucrose esters of fatty acids including cis and trans isomers; or (5) di- and triglycerol esters with carbon chain lengths from two to twenty-four.

Although fatty acids that are esterified to monohydric alcohols are fairly inactive as microbicides in the present invention, certain fatty acids esterified to a polyhydric alcohols typically become more active. For example, lauric acid is perhaps a preferred fatty acid from a point of view of activity of medium-chained fatty acids; however, it is noted that it can cause irritation of the skin on repeated use when used in conjunction with alcohols, for example a 70% solution of n-propanol. When lauric acid is esterified to glycerol to form a monoester therewith, but not the di- or tri- esters, it has been found to have a high activity. Functioning, as an emollient, it retards the evaporation of alcohol and is also no longer irritating to the skin. For this reason the glycerol monolaurate is one preferred persistent component.

As seen with the quick kill alcohols, the fatty acids and fatty acid esters also tend to increase in antimicrobial activity as the pH of the composition decreases. Typically, as the pH of a solution containing the fatty acids decreases and/or as the chain length of the fatty acid increases, the microbicidal activity of the fatty acid also increases. Long-chain unsaturated fatty acids are more effective against gram-positive microorganisms at a neutral pH. However, long-chain unsaturated fatty acids as well as the medium-chain saturated fatty acids and the lauric acid ester of glycerol are active against gram-negative bacteria as the composition becomes more acidic, especially in the presence of a calcium and magnesium chelator. Preferred calcium and magnesium chelators are citric acid, phosphoric acid, phosphonic acids and polyphosphoric acids at an acid pH, or sodium hexametaphosphate, sodium tripolyphosphate or EDTA at neutral pH.

For the best activity, it is necessary for the fatty acids to be free fatty acids in solution. An anionic, cationic or non-ionic surfactant may impair the free fatty acid performance in microbicidal activity. The alcohols typically are good solvents for the free fatty acids. It is possible to add iodine to this solution to further improve the bactericidal effects of the solution; however, when unsaturated fatty acids are utilized, it is necessary to add excess iodine and iodide to account for the halogenation of the unsaturated fatty acids sites by the iodine. This is accomplished by the addition of iodide in a ratio of at least two equivalent weights of iodide to iodine.

The preferred quick-kill and persistent lipid components of the present invention for intact skin include medium-chain saturated fatty acid monoesters, long-chain unsaturated free fatty acids, and long-chain unsaturated fatty acid polymers. The preferred range of the medium-chain fatty acid esters is from about 0.001% to 7% by weight; and more preferred, from about 1% to about 3% by weight. The preferred medium-chain fatty acid monoester is glycerol monolaurate (Lauricidin). The range for the unsaturated fatty acid is from about 0.0001% to about 30% by weight, the preferred range is from about 0.01% to about 10% by weight, and the most preferred range is from about 1% to about 50% by weight.

The preferred lipid components of the present invention for application to the eyes, ears, and vagina would include the short-chain free fatty acids in combination with the medium-chain fatty acids and long-chain fatty acids. The desirable range of the short-chain fatty acids is from 0.01% to 15% by weight and preferably from 0.1% to 10% by weight.

Further persistent components may be added to the antiseptic composition of the present invention: oils, preservatives, and/or nitrogen compounds including pyridinethiones.

Oils, with their inherent lipophilic, hydrophobic character could function as a lipid persistent component in the present invention. This would include: safflower oil, sesame seed oil, wheat germ oil, evening primrose, soybean oil, canola oil, tea tree oil (Lelaleuca alternifolia), eugenol, isoeugenol, thyme (White and Red), cinnamon, bay oil, linseed oil, and borage oil.

Certain preservatives may be added to the antiseptic composition as highly effective quick-kill and/or persistent components. These include preservatives that are conventionally utilized in the cosmetics industry: 1) acids and phenolics such as benzoic acid and salts, acetic acid and salts, sorbic acid and salts, propionic acid and salts, lactic acid and salts, boric acid and salts, dehydroacetic acid, sulphurous and vanillic acids, phenol, cresol, chlorocresol, o-phenylphenol, chlorothymol, parabens (alkyl esters of parahydroxy-benzoic acid such as methyl-, ethyl-, propyl-, butyl- and benzyl-p-hydroxy benzoates); 2) mercurials such as thimerosal, phenylmercuric acetate and nitrate, nitromersol, and sodium ethylmercurithiosalicylate; 3) aromatic alcohols such as benzyl alcohol, beta-phenylethyl alcohol, phenylethyl alcohol (especially effective against gram-negative organisms, e.g., Pseudomonas), and phenoxy-2-ethanol; 4) imidazolidinyl urea, or Germall 115, and diazolidinyl urea, or Germall II (Sutton Laboratories, Inc., Chatham, NJ); 5) oils such as p-cymene, linalool, geraniol, nerol, thymol, carvacrol, eugenol, isoeugenol, safrole, benzaldehyde, cumic aldehyde, cinnamic aldehyde, salicylaldehyde, pulegone, thujone, ascaridole, and cineol; 6) miscellaneous agents such as chlorhexidine, chloroform, bronopol, glydant, and a mixture of 5-chloro-2-methyl-3-(2H)isothiazolone and 2-methyl-3-(2H)isothiazolone known as Kathon CG (Rohm & Haas, Philadelphia, PA); and 7) combinations of above.

The bispyrithiones, such as zinc pyrithione and alkaline earth metal salts of bispyrithione are chosen for their broad-spectrum of activity and outstanding persistence. Zinc pyrithione is not easily absorbed through intact epidermis or mucous membranes, but is soluble in sebum and penetrates into hair follicles, thereby resisting removal by rinsing. Because of its solubility in sebum this compound is very useful in acne vulgaris, acne rosacea, and as a deodorant. It decreases the cell turnover rate in hyper proliferative dermatoses such as psoriasis and seborrhea. Because of its broad-spectrum, including fungi, this component would be useful in athlete's foot, tinea versicolor, tinea cruris, candidiasis, diaper rash, and onychomycosis.

Bispyrithione salts (also called pyridinethione salts) are highly effective as components of the present invention, including zinc and magnesium salts of bispyrithione. Unfortunately, these preferred bispyrithione salts are basically insoluble in alcohol. Consequently, it has been difficult to produce a composition using a bispyrithione in conjunction with a quick-kill component of the present invention, especially the alcohols, where the quick-kill component(s) and persistent component(s) can be incorporated in a single, stable solution.

It has also been found that magnesium pyrithione (Omadine MDS) cannot readily be used as the persistent component with the fatty acids of the present invention because an insoluble precipitate forms that reduces the activity of the free fatty acids and bispyrithione. However, magnesium pyrithione would be useful in formulas which do not incorporate fatty acids. Magnesium pyrithione would be very useful in applications for the eyes. The potent activity of this compound would allow very small concentrations to be used, for example, as low as ten parts per million. Preferable concentrations would range from about 0.0001% to about 1.0%.

In lotion or cream formulations, zinc pyrithione (Zinc Omadine) is compatible with free fatty acids as the zinc molecule is chelated by the pyrithione and unavailable to couple with the free fatty acids. Zinc Omadine may be added to the formula in the presence of long-chain alcohols, long-chain unsaturated fatty acids, medium-chain saturated fatty acids, and/or their esters with an emulsifier. Zinc Omadine can be incorporated at a concentration of about 0.0001% to about 5.0% (w/v), more preferably in a range of from about 0.5% to about 4% (w/v), and most preferably from about 1.0% to about 2.0% (w/v).

It has been surprisingly found that bispyrithione (Omadine Disulfide) is highly soluble in the solutions suggested for the quick-kill component, especially alcohol. For example, when bispyrithione is added in about 2.5% to 3% (w/v) to a solution of Emery 644 fatty acids in a 70% (v/v) n-propanol solution, bispyrithione fully dissolves and substantially maintains full activity of both the fatty acids and bispyrithione. That bispyrithione would dissolve in the n-propanol and fatty acid solutions is unexpected. Bispyrithione, when added as the persistent component of the present invention, is preferably added in a range of about 0.0001% to about 5.0% (w/v), more preferably from about 0.5% to about 4% (w/v), and most preferably from about 1.5% to about 2.5% (w/v). Bispyrithione is effective against all microorganisms.

Other ingredients may be added to improve specific types of antiseptic formulations. For example, it is foreseen that glycyrrhetinic acid would be preferably added to skin formulations to act as an anti-inflammatory agent. The addition of hyaluronic acid to the antiseptic would be beneficial in wound and burn antiseptics. For treatment of viruses, 2-deoxy-D-glucose may be added to increase the antiviral activity of the antiseptic.

The antiseptic may also utilize various penetration controlling agents to control the degree to which the antiseptic composition penetrates through the stratum corneum, epidermis and dermal layers. For the antiseptic to permeate throughout the epidermis, the preferred compositions of the present invention may utilize a membrane penetration component(s). These components helps to ensure that the microorganisms that are hidden in the pilosebaceous glands, sweat ducts and in the superficial and deep layers of the stratum corneum are also killed with the surface treatment. Consequently, preferred membrane penetration compounds are also microbicidal. Some of the preferred penetrating compounds are unsaturated long-chain fatty acids, esters of fatty acids, propylene glycol, medium-chain saturated fatty acids and medium-chain alcohols (C8 through C14). When it is important to inhibit penetration beyond the basal cell layer this is easily accomplished by incorporating combinations of mineral oil, petrolatum, or cetyl and stearyl alcohols. When it is important to prohibit irritants from contacting the skin, this may be accomplished by incorporating polymers of linoleic acid.

Improved kill of microorganisms is also found if a chelating agent component is incorporated. Preferably the chelating agent is a calcium and magnesium chelating agent that also tends to lower the pH of the composition. Suitable chelating agents of this type include polyphosphoric acid, citric acid, phosphonic acids and phosphoric acids. Useful chelating agents include: aminopolycarboxylic acids (such as hydroxyethyl imino diacetic acid, nitrilo triacetic acid, ethylene diamine tetraacetic acid, hydroxyethyl ethylenediamine triacetic acid, and diethylene triamine pentacetic acid); alpha-hydroxy acids (such as tartaric acid, citric acid, and gluconic acid); and condensed phosphates and phosphonates. The preferred chelating agents for use in the present invention are dependent on the final use. At an acid pH, phosphoric or phosphonic acids are preferable. At neutral pH, either a mixture of phosphonic acids and their salts, or ethylenediamine tetraacetic acid and pharmaceutically acceptable salts thereof, especially sodium ethylene diamine tetraacetate are preferable. Other useful calcium chelating components include ethane-1-hydroxy-1,1'-diphosphonic acid, methane diphosphonic acid, hydroxy methane diphosphonic acid and mixtures thereof.

It is also preferred to incorporate an antioxidant component into the compositions of the present invention to prevent saturation of the unsaturated fatty acids. Suitable antioxidants include betahydroxy toluene, Vitamin E, Vitamin C, alpha-tocopherol, and propyl gallate or mixtures containing propyl gallate such as Tenox PG or Tenox S1 (Eastman Chemical Co., Kingsport, TN). A preferred antioxidant is propyl gallate which may be increased above antioxidant levels to achieve a potent anti-inflammatory effect. The gallic acid esters, especially methyl gallate, also may be used to exert an anti-viral effect, especially on the herpes virus and cytomegalovirus. The gallic acid esters may also function to prevent damage to the skin from ultraviolet light or radiation damage.

Antiseptic products that are intended for chronic use can be detrimental to the skin, particularly agents that are frequently applied to the hands, e.g., health care personnel hand washing agents. Cetyl and stearyl alcohols are common components of lotions and creams. When cetyl and stearyl alcohols are added to the n-propanol antiseptic formula they precipitate at relatively cool ambient temperatures, making them unsuitable. However, if cetyl and stearyl alcohols are added when ethoxylated, then a stable solution can be obtained. Particularly preferred products are the emulsifying waxes Polawax or Polawax 31 (Croda, Inc., Parsippany, NJ), or ethoxylated fatty alcohols such as Eumulgin B2 (Henkle Corporation, Emery Group, Cincinnati, OH). When Polawax is added up to a concentration of four percent in alcohol the antiseptic formulation remains clear. When Polawax is added in increasing amounts with lipids, the antiseptic becomes an emulsion.

Particularly preferred are Polawax concentrations of six to twelve percent. When excess Polawax is added, it causes the antiseptic to become very viscous or even a solid. When appropriate amounts of Polawax are used, the emulsion provides a mechanism of using previously insoluble ingredients, e.g., Zinc Omadine which may be suspended into a stable emulsion. The cosmetic appeal and activity may be significantly enhanced by using long-chain alcohols or esters in an emulsion formulation. Polymers of long-chain unsaturated fatty acids may be used to produce anti-irritation effects.

A coloring and texture component may also be utilized within the composition for different embodiments. In particular, a thickener may be utilized for certain embodiments to decrease the quick evaporation of alcohols especially when used as the quick-kill component, and to keep various low viscosity fluids from quickly running off of the surface being treated. An effective thickener has been found to be hydroxypropyl cellulose sold under the trademark Klucel by Aqualon, Wilmington, DE. When the compositions are utilized for surgery, dyes may be incorporated such that the area treated by the antiseptic may be quickly visualized by the medical practitioners to ensure application. In some of the embodiments a formulation may be varied so as to effectively modify the composition into a cream for certain purposes. The components of the present invention may be made mucoadhesive with sodium carboxymethyl cellulose, polyethylene oxides such as Polyox WSR 301, propylene glycol, and polyethylene glycol 8,000 to make a gel. This would facilitate treatment for lesions in the mouth, such as aphthous ulcers. Also, this combination would be an effective antiseptic to apply to the periurethral area to prevent nosocomial infections associated with a chronic indwelling Foley catheter. Certain vaginal applications would be enhanced with the addition of mucoadhesive components.

The antiseptic compositions of the present invention may also be combined with conventional over-the-counter (OTC) or prescription ingredients to achieve salutary effects. Current OTC treatment for acne would include salicylic acid, sulfur, resorcinol, resorcinol monoacetate, and benzoyl peroxide, while agents restricted to prescription, e.g., antibiotics include trinetoin, erythromycin, tetracycline, clindamycin or their combinations. Current treatment for topical fungal infections including undecylenate, miconazole, clotrimazole, tolnaftate, terconazole, and butoconazole may be combined with ingredients of the present invention. Current OTC treatment for the prevention of sunburn include para-aminobenzoic acid, ethylhexyl p-methoxycinnamate, and cinnamate. The ingredients of the present invention may be combined with agents for minor wound care including OTC agents such as neomycin, bacitracin, polymyxin B, and Neosporin, or antibiotics. Other combinations would include product enhancing agents for a soothing, cooling, detergent, astringent, antipruritic effect or anti-inflammatory agents (OTC or prescription strength corticosteroids). The ingredients of this invention may be combined with anti-metabolites, cytostatic agents, keratolytic agents, or tar products. The compounds of this invention may be combined with OTC or prescription eye and ear ingredients.

While low pH is a distinct advantage in quick kill, it is foreseen that too low a pH could be detrimental in specific formulations of the antiseptic compositions of the present invention. For example, too low a pH could cause hydrolysis of the thickener (Klucel), ester formation of the fatty acids, and the glycerol monolaurate (Lauricidin).

Antiseptic compositions of the present invention have been are formulated to provide the following advantages: are antimicrobial, both immediately and long-term; provide a very high ratio of kill, preferably a kill to zero; provide a broad spectrum of antimicrobial kill; are not irritating to skin of humans and mammals and can be readily adapted for usage on other tissues; are adapted to penetrate and bind into the epidermis and kill microorganisms therein; are adapted to bind to the skin surface and kill microorganisms therein; have substantial persistence both in terms of maintaining a low quantity of microorganisms on tissue treated by the compositions for a substantial period of time and not allowing the level of microorganisms on or in the tissue to return to normal for a substantial period of time; are user-friendly, having odor, tactile qualities and other characteristics which are pleasant to those both applying and receiving the compositions; are adaptable or modifiable to a wide range of medical uses as antiseptics including use on sensitive tissues; and are relatively easy to prepare, stable in shipment and especially effective for their intended purposes.

### EXAMPLE 1: PREPARATION OF A MINIMUM REQUIREMENT ANTISEPTIC WITHOUT FATTY ACIDS

To prepare the antiseptic as indicated in Table I, initially mix approximately 95% of the volume of n-propanol with the thickening agent Klucel. To hydrate the Klucel, stir for 2-3 hours with equipment that produces high shear, or overnight with equipment that only produces low shear. Add the additional 5% of n-propanol, and while continuously stirring, add the appropriate amount of the emulsifier. Adjust the pH as required, add the appropriate amount of Zinc Omadine, and q.s. to 100% with deionized water.

**Table I.**

| EXEMPLARY FORMULATION FOR MINIMUM REQUIREMENT ANTISEPTIC | |
|---|---|
| COMPONENT | CONCENTRATION(%) |
| ALCOHOL | |
| N-Propanol^{a} | 70 |

| EMULSIFIER | |
|---|---|
| Polawax^{b} | 6 |

| PRESERVATIVE | |
|---|---|
| Zinc Omadine^{b,c} | 2 |

| THICKENING AGENT | |
|---|---|
| Klucel^{b} | 1 |

| pH ADJUSTING AGENT | |
|---|---|
| Phosphoric acid^{b} | 2 |

| | |
|---|---|
| ^{a} Concentration in percent (v/v). | |
| ^{b} Concentration in percent (w/v). | |
| ^{c} Quick-kill and persistent agent. | |

### EXAMPLE 2: PREPARATION OF MINIMUM REQUIREMENT ANTISEPTIC WITH FATTY ACIDS

To formulate an antiseptic containing fatty acids, the ingredients given in Table II, initially mix approximately 95% of the volume of n-propanol with the thickening agent Klucel. To hydrate the Klucel, stir for 2-3 hours with equipment that produces high shear, or overnight with equipment that only produces low shear. Add the additional 5% of n-propanol, and while continuously stirring, add the appropriate amount of the emulsifier, fatty acid, and antioxidant. Adjust the pH as required, add the appropriate amount of Zinc Omadine, and q.s. to 100% with deionized water.

**Table II.**

| EXEMPLARY FORMULATION FOR MINIMUM REQUIREMENT ANTISEPTIC | |
|---|---|
| COMPONENT | CONCENTRATION(%) |
| ALCOHOL | |
| N-Propanol^{a} | 70 |

| EMULSIFIER | |
|---|---|
| Polawax^{b} | 6 |

| FATTY ACID | |
|---|---|
| Emery 305^{b} | 5 |

| PRESERVATIVE | |
|---|---|
| Zinc Omadine^{b},^{c} | 2 |

| THICKENING AGENT | |
|---|---|
| Klucel^{b} | 1 |

| pH ADJUSTING AGENT | |
|---|---|
| Phosphoric acid^{b} | 2 |

| ANTIOXIDANT | |
|---|---|
| Propyl gallate^{b} | 0.1 |

| | |
|---|---|
| ^{a} Concentration in percent (v/v). | |
| ^{b} Concentration in percent (w/v). | |
| ^{c} Quick-kill and persistent agent. | |

### EXAMPLE 3: IN VITRO MEASUREMENT OF ANTIMICROBIAL ACTIVITY OF OMADINE AND OTHER POTENTIAL COMPONENTS OF NEW ANTISEPTIC FORMULATIONS

Seven compounds or mixtures were tested including bispyrithione (Zinc Omadine), two free fatty acid hydrolysates (Emery 315 and Emery 644), acetic acid, propylene glycol, and two antiseptic formulations that contained 2.5% bispyrithione and 3.0 % fatty acids, with or without 0.25% Klucel (AS+K and AS-K, respectively). The pH of the antiseptic mixtures was 2.7-2.8.

Over 200 bacterial and fungal isolates from clinical patient infections at the University of Iowa Hospitals and Clinics (Iowa City, Iowa) were used to determine antimicrobial activity. Each strain was identified by routine methods (Vitek Systems, API, etc.) in use in the Medical Microbiology Division laboratories. The following species were processed: *Staphylococcus aureus* (10 strains, five resistant to methicillin), coagulase-negative staphylococci (10 strains representing four species, five resistant to methicillin), *Enterococcus* spp. (10 strains), *Streptococcus pyogenes* (10 strains), beta-hemolytic streptococci groups B, C, and G (10 strains), *Corynebacterium jeikeium* (10 strains), *Corynebacterium parvum* (previously *Propionibacterium acnes)* (10 strains), *Escherichia coli* (10 strains representing two species), *Enterobacter* spp. (10 strains), *Klebsiella* spp. (10 strains, representing two species), *Pseudomonas aeruginosa* (10 strains), *Citrobacter* spp. (10 strains), indole-positive *Proteeae* (10 strains), *Salmonella*/*Shigella* spp. (10 strains), *Serratia marcescens* (10 strains), *Acinetobacter* spp. (10 strains), *Xanthomonas maltophilia* (10 strains), *Prevotella bivia-disiens* (10 strains), *Bacteroides fragilis* (10 strains), *Gardnerella vaginalis* (10 strains), *Lactobacillus* spp. (10 strains), *Mobiluncus* spp. (10 strains), *Aspergillus* spp. (5 strains), *Candida albicans* (5 strains), Candida spp. (5 strains), and dermatophytes (5 strains).

All susceptibility testing was performed by methods conforming to the recommendations of the National Committee for Clinical Laboratory Standards (NCCLS). Mueller-Hinton agar (Difco Laboratories, Detroit, MI) was used with supplemental 5% sheep erythrocytes for fastidious species (streptococci, corynebacteria). Dilution schedules were selected based on preliminary pilot studies covering a dilution range of 10% to 0.001% solutions of each compound or mixture. The following dilution ranges were used for each substance/mixture following pilot study experiments: 1) bispyrithione, 0.25% to 0.001%, or 250 to 1 microgram/ml; 2) acetic acid and the two antiseptic mixtures, 4% to 0.015%; and 3) propylene glycol and two fatty acid hydrolysates, Emery 644 and Emersol 305, 16% to 0.25%. Note that the antiseptic solutions and fatty acids were listed as % solutions but actually represent dilutions of the provided formulation(s), e. g. 1:25 to 1:6400.

The pH of the agar medium was 7.2 to 7.4 conforming to the NCCLS recommendations. Therefore, the antimicrobial action of several of these substances which perform maximally at acidic pH ranges was potentially underestimated because of testing at standard microbiology pH levels favoring the growth of pathogenic organisms.

Table III lists the minimum inhibitory concentrations (MICs) obtained in the pilot study in which 10% to 0.001% solutions were tested against laboratory strains of seven bacteria and one yeast. From these results, the assay dilution ranges were selected.

When the antiseptic formulations (2.5% Zinc Omadine and 3.0% fatty acids in n-propyl alcohol) with and without a thickening agent (0.25% Klucel) were tested as a dilution of the final concentration, excellent inhibition was observed as shown in Table III. All gram positive organisms were inhibited at less than 0.1% dilution, and all gram negative organisms, at less than 1.0% dilution.

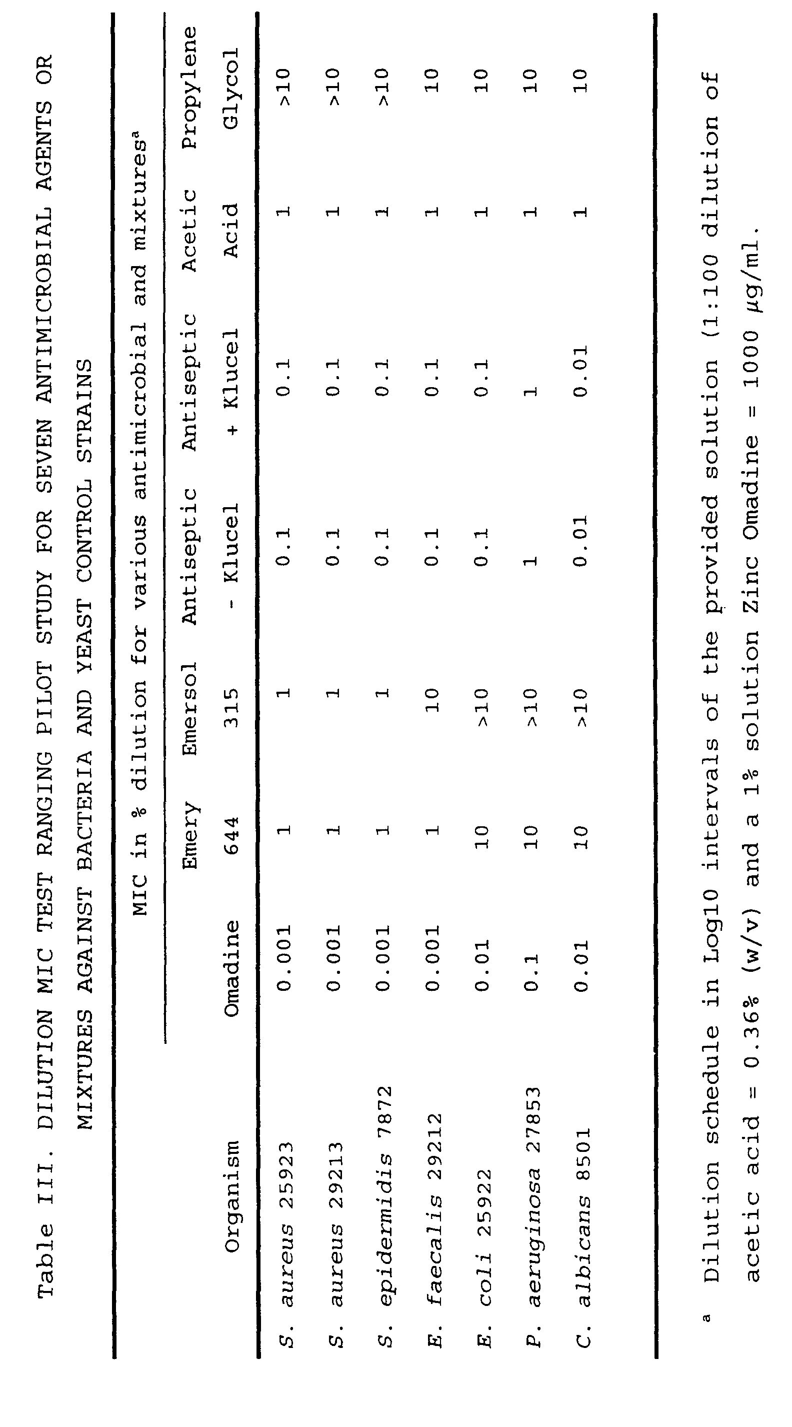

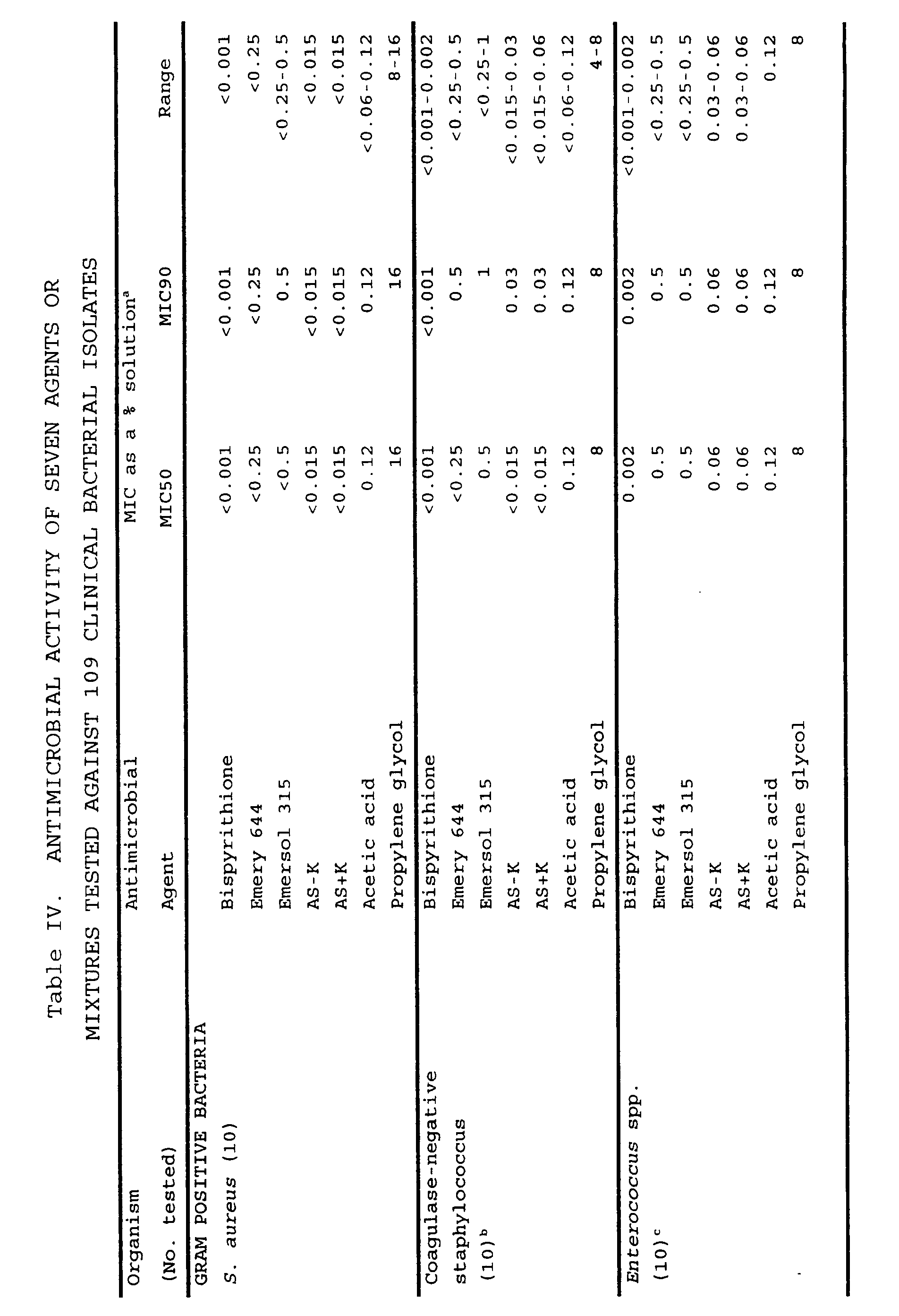

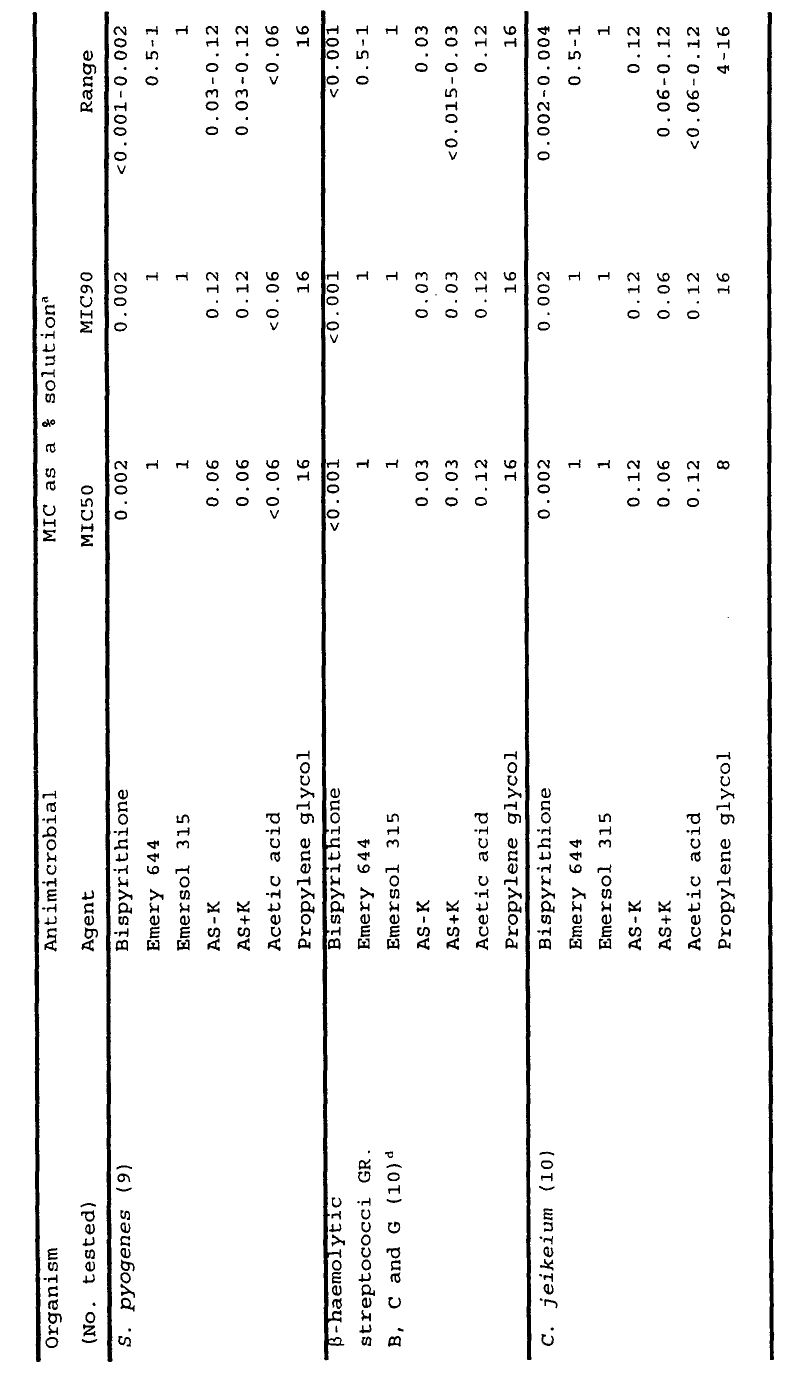

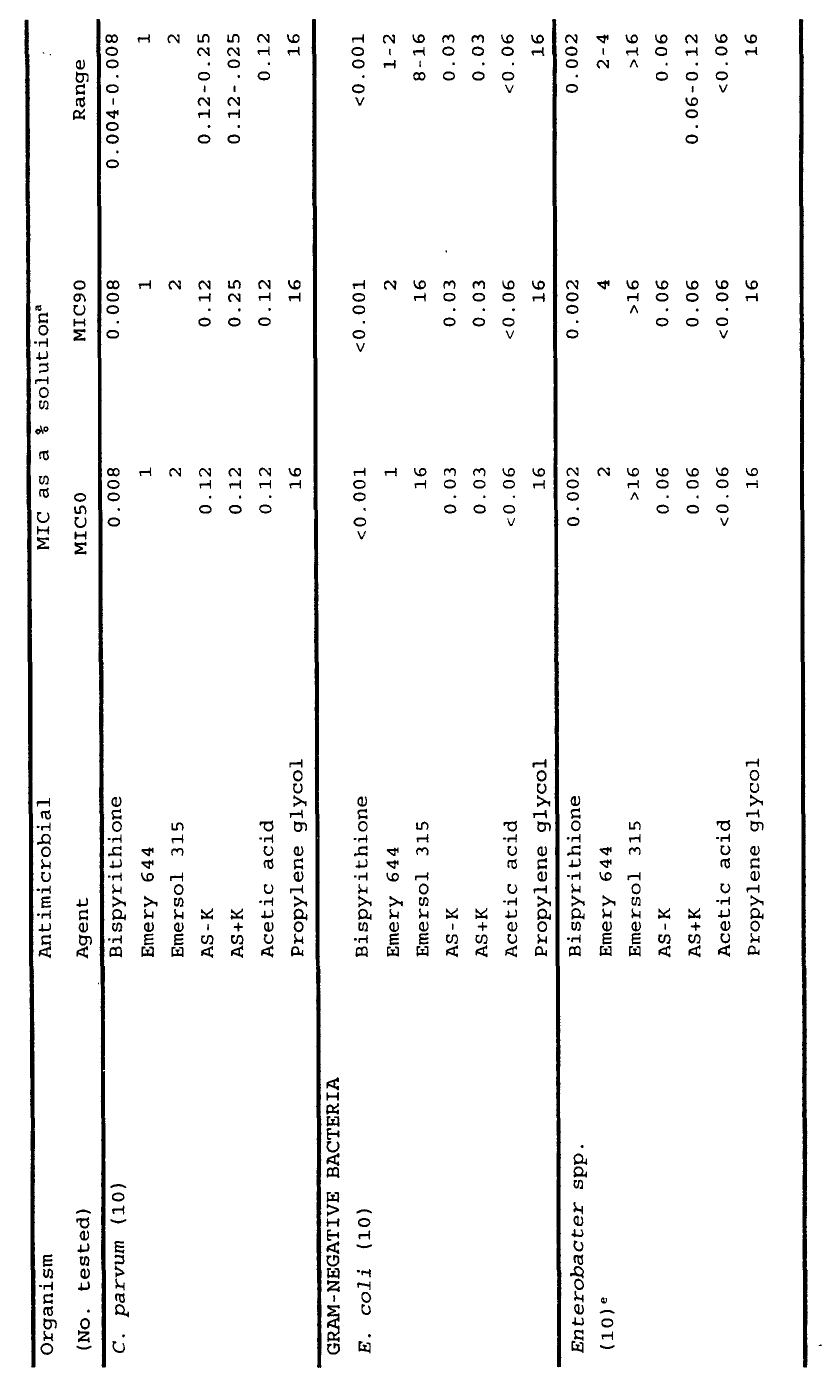

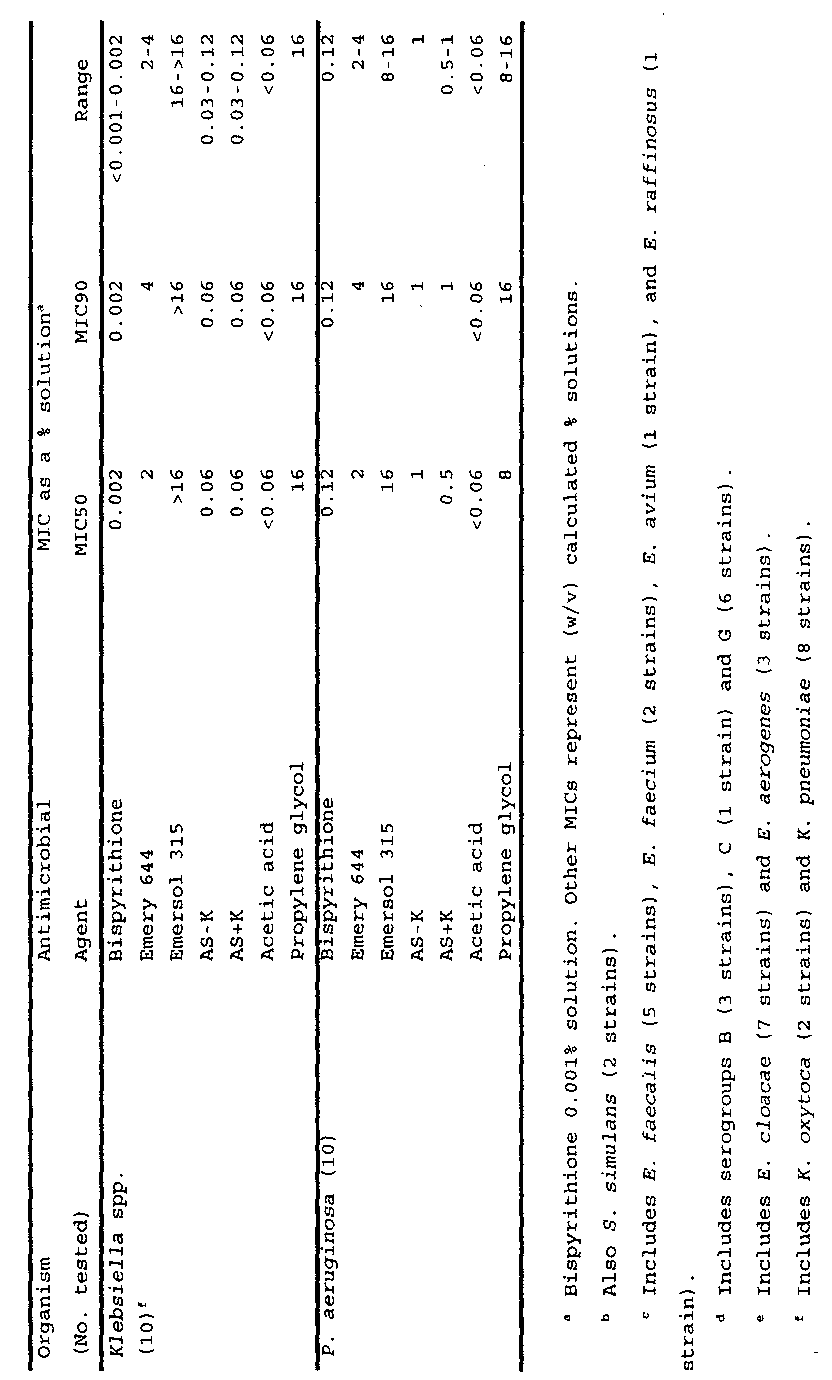

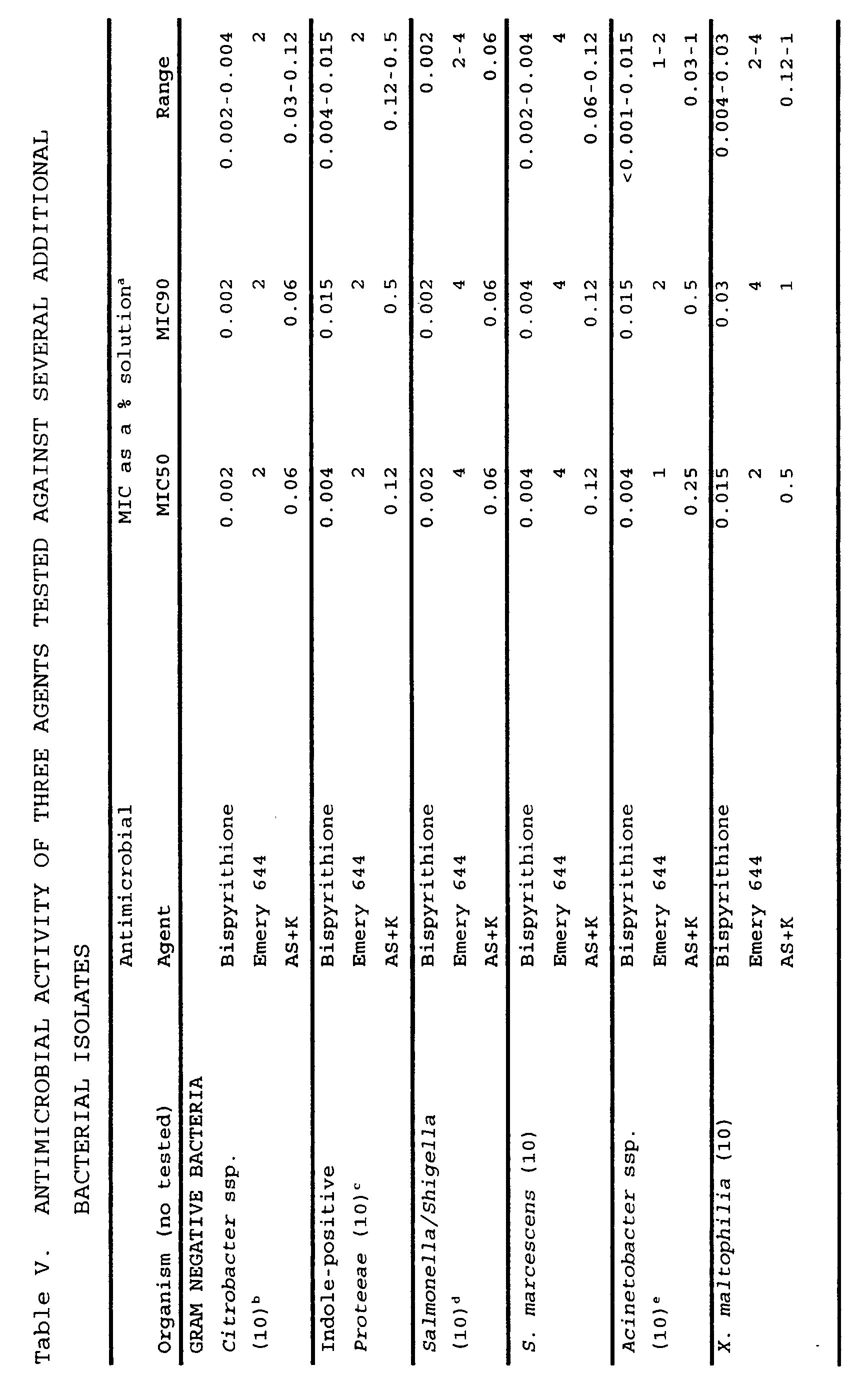

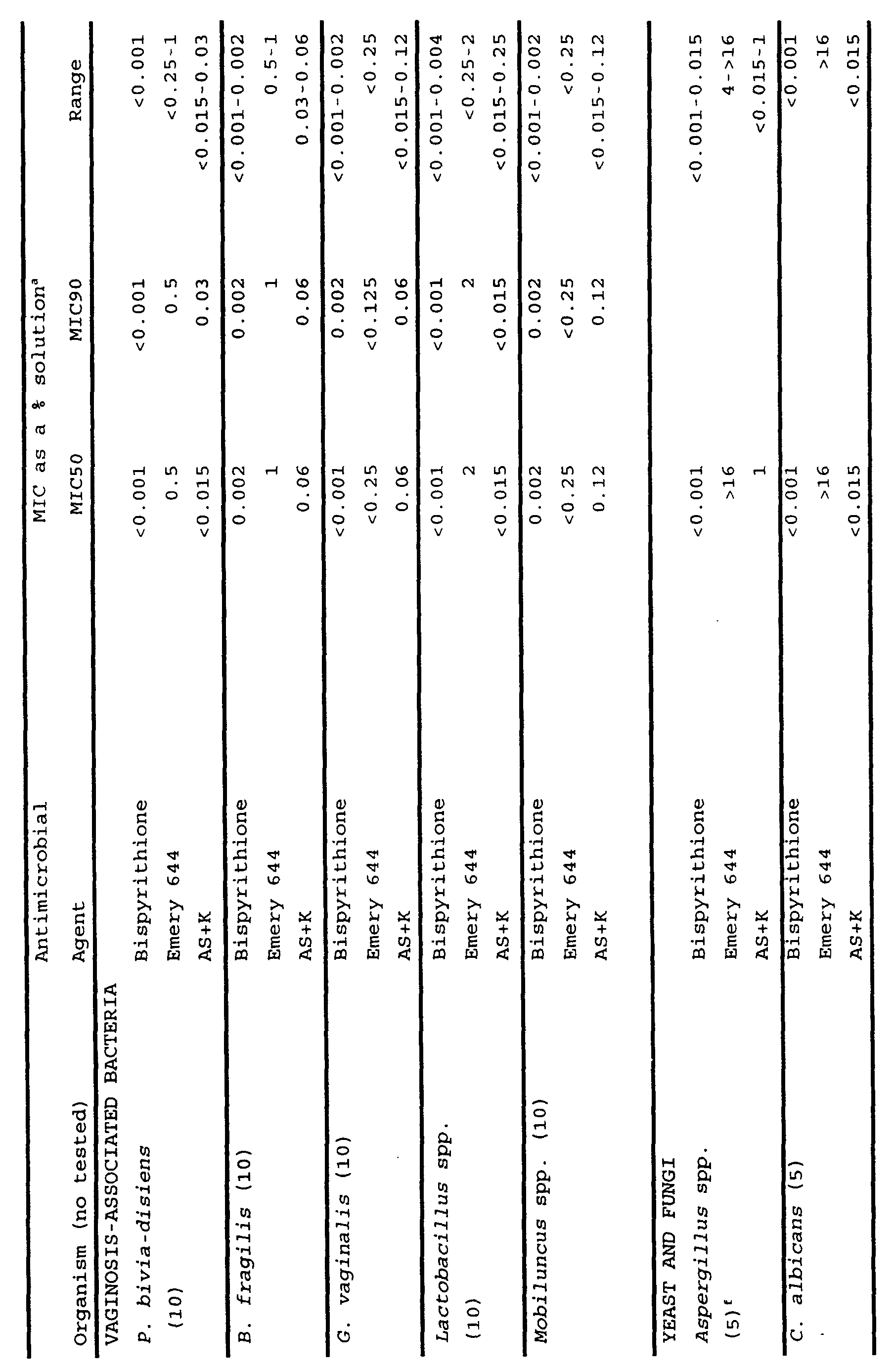

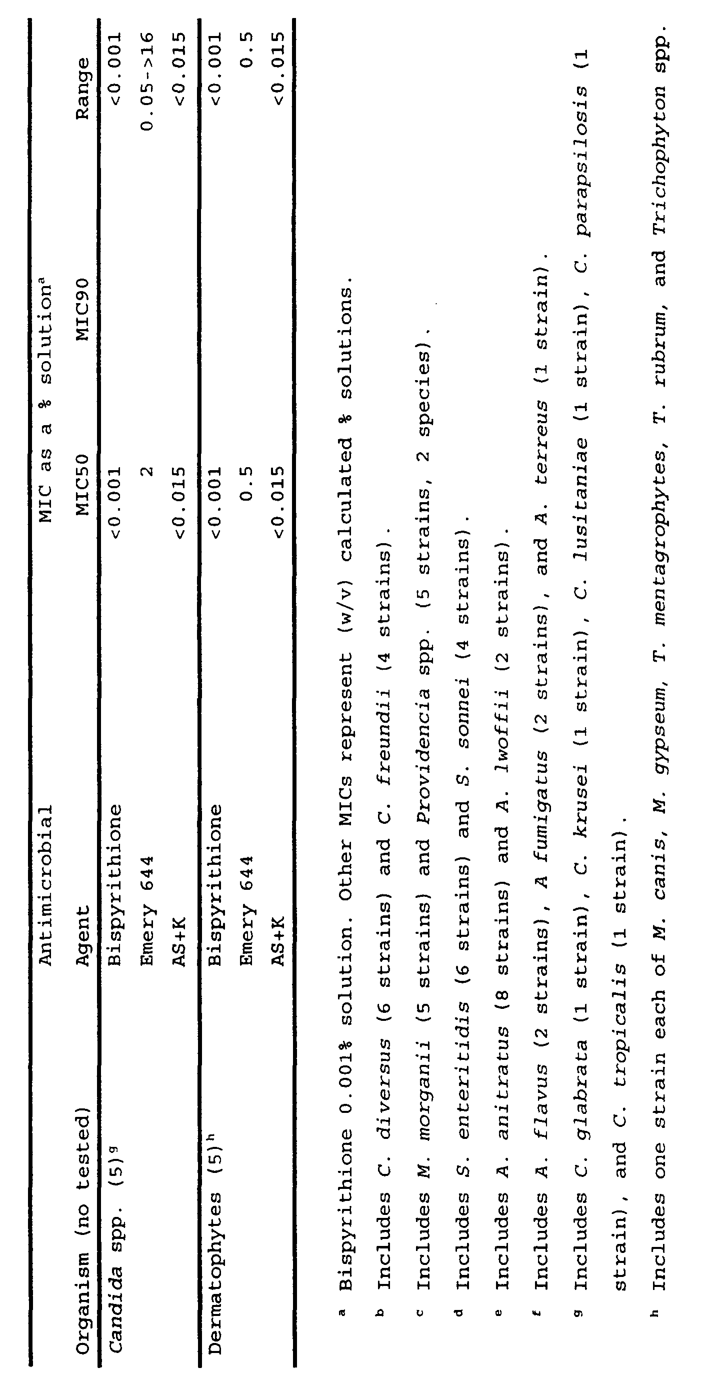

**Table VI.**

| EFFECTS OF 5% SHEEP BLOOD ON THE AGAR DILUTION MICs OF THE ANTISEPTIC SOLUTION PLUS KLUCEL | | |
|---|---|---|
| | MIC as a % Antiseptic Solution: | |
| Organism | Agar + 5% Sheep^{a} | Agar Alone |
| *C. albicans* (8501) | 0.008 | 0.015 |
| *E. coli* (25922) | 0.015 | 0.015 |
| *E. faecalis* (00049) | 0.015 | 0.015 |
| *P. aeruginosa* (27853) | 0.25 | 0.25 |
| *S. aureus* (29213) | 0.03 | 0.03 |
| *S. aureus* (25923) | 0.03 | 0.03 |

| | | |
|---|---|---|
| ^{a} Agar supplemented with 5% sheep blood. | | |

Results for seven antimicrobial agents against a wide variety of the clinical bacterial isolates were tabulated as the concentration inhibiting 50% and 90% of the tested organisms and are presented in Table IV. The range of MICs is also listed for each tested substance.

Antimicrobial activities of bispyrithione, fatty acids, and antiseptic plus Klucel were measured using clinical isolates of several microorganisms, including additional gram positive bacteria, vaginosis-associated bacteria, yeasts and molds. Table V presents the results as the concentration inhibiting 50% and 90% of the tested organisms, with the MIC range also provided.

These in vitro susceptibility tests using NCCLS procedures confirm the spectrum and level of potency previously stated by the bispyrithione manufacturer (Olin Chemicals). Bispyrithione has a spectrum of activity inhibiting all significant gram positive pathogens at <0.004%, or <40 micrograms/ml. Similarly, with the exception of *Pseudomonas* and *Xanthomonas* spp., all enteric bacilli tested had MIC's at <0.004%, or <40 micrograms/ml. *Pseudomonas aeruginosa* and *Xanthomonas maltophilia* had higher MICs at <0.12% (120 micrograms/ml) and 0.15% (150 micrograms/ml), respectively. *Aspergillus* spp., *Candida albicans,* other *Candida* spp., and the dermatophytes were inhibited at a bispyrithione concentration of <0.001%, or 10 micrograms/ml.

The fatty acid hydrolysates had a comparable spectrum of activity to that of bispyrithione. The Emery 644 was generally equal to or 2-fold superior in potency to Emersol 315 when tested against gram positive organisms. All gram positive bacteria tested were inhibited by the 1:50 dilution of the original hydrolysate. Emery 644 was also more active than Emersol 315 versus gram negative bacilli (4- to >8-fold) and *Candida albicans.* All Emery 644 MIC's were <4%, or a 1:25 dilution of the provided full-strength hydrolysate.

When the antiseptic formulations of 2.5% bispyrithione and 3.0% fatty acids in propyl alcohol with and without Klucel were tested as a dilution of the final concentration, excellent inhibition was observed. All gram positive and gram negative organisms were inhibited at <0.12% (1:800 dilution) and <1% (<1:100 dilution), respectively. Vaginosis-associated organisms were effective at <0.025% (<1:400 dilution), and fungi at <1% (<1:100 dilution).

To assess the effect of blood on the antimicrobial activity of the antiseptic solution consisting of 2.5% bispyrithione and 3.0% fatty acids in n-propyl alcohol plus Klucel, the NCCLS procedure was performed using Mueller Hinton agar plates with and without 5% sheep blood. Antimicrobial activity was measured as the concentration inhibiting 50% of the tested organisms. As shown in Table VI, comparisons of MICs for the antiseptic with Klucel determined on media with and without 5% sheep erythrocytes failed to demonstrate any significant differences. Small amounts of blood appear to not affect the potency of fatty acids or the bispyrithione.

### EXAMPLE 4: PREPARATION OF HEALTH CARE PERSONNEL HAND WASH

Four formulations of health care personnel hand wash antiseptic were prepared according to the procedure given in Example 2 by combining the ingredients given below in Table VII, adjusting the pH to 3.00-3.50 with either phosphoric acid, Dequest 2010 or citric acid, and q.s. to 100% with deionized water. The final concentrations are indicated either as percent (v/v) or percent (w/v).

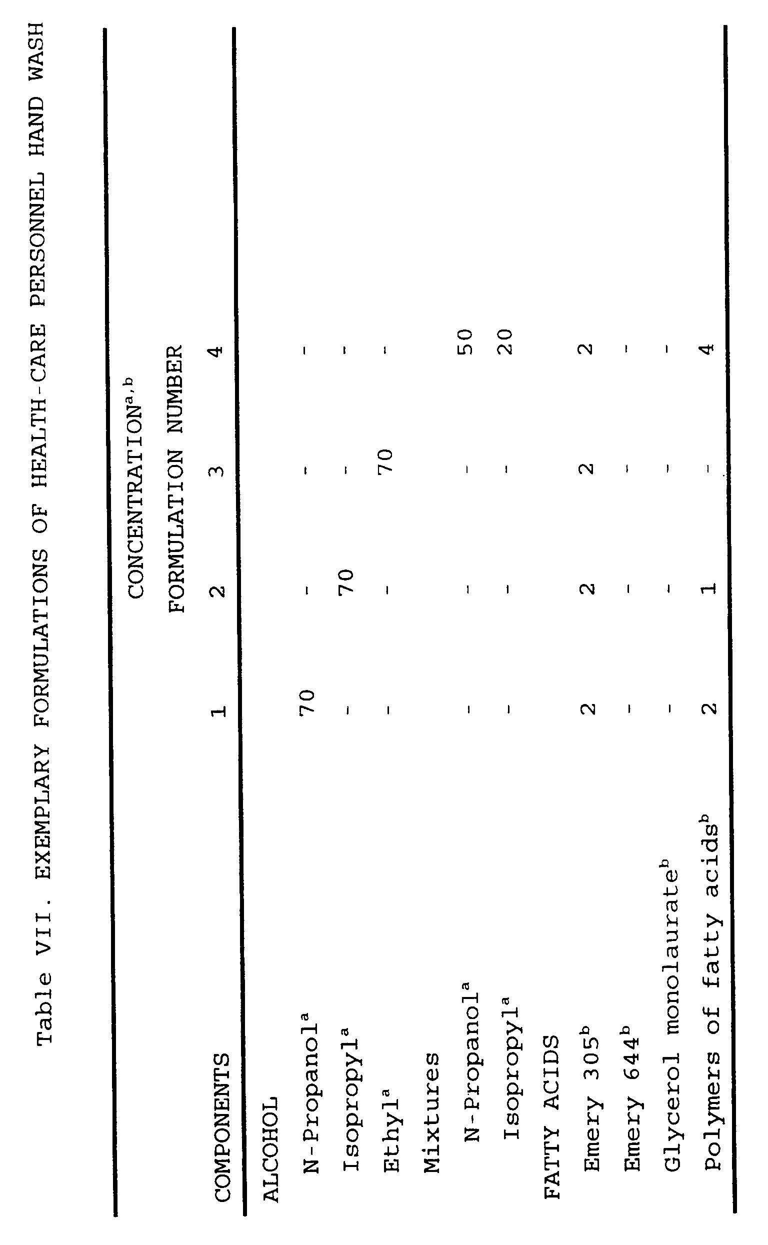

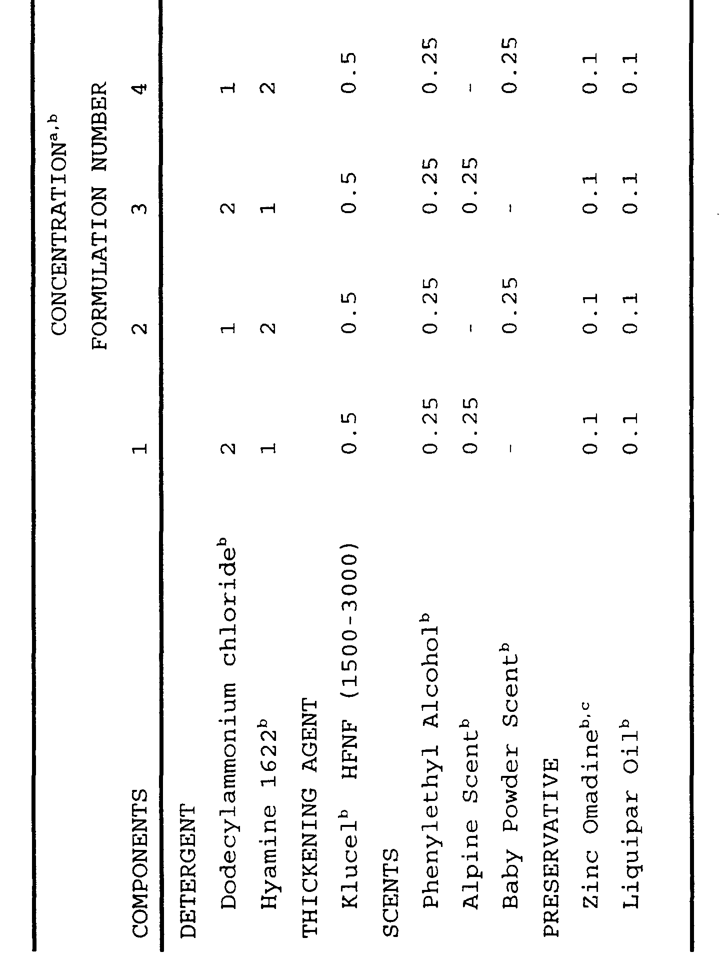

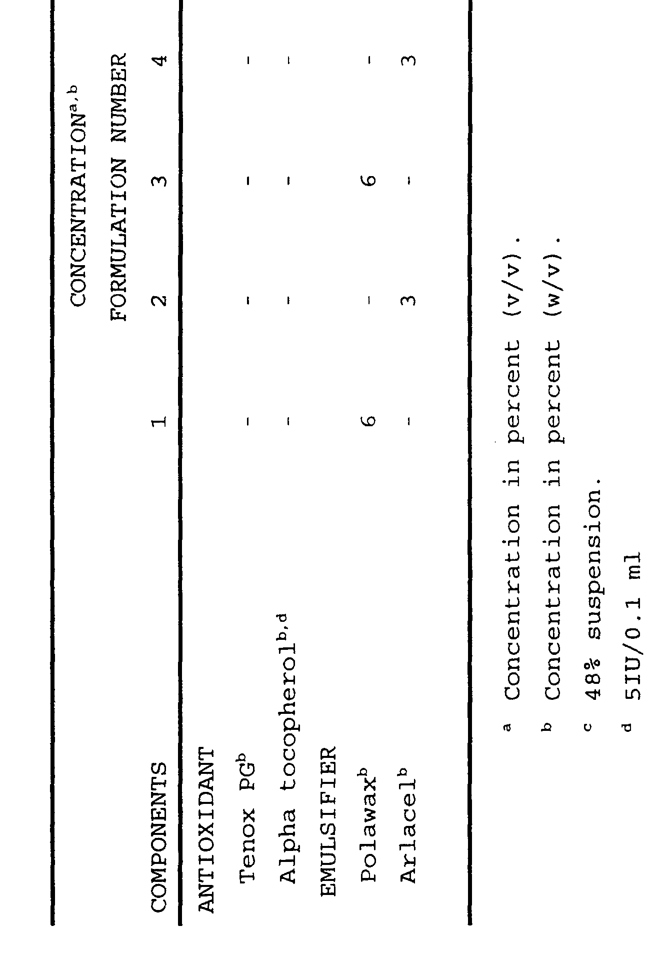

### EXAMPLE 5: PREPARATION OF SURGICAL HAND SCRUB

To provide stronger cleansing action and deeper penetration of the antiseptic action preferred in pre-operative hand scrubbing, four formulations of surgical hand scrubs were prepared by combining the ingredients given below in Table VIII, adjusting the pH to 3.00-3.50 with phosphoric acid, and q.s. to 100 % with deionized water. The final concentrations are indicated either as percent (v/v) or percent (w/v).

**Table VIII.**

| EXEMPLARY FORMULATIONS OF SURGICAL HAND SCRUB | | | | |
|---|---|---|---|---|
| | CONCENTRATION^{a,b} FORMULATION NUMBER | | | |
| COMPONENTS | 1 | 2 | 3 | 4 |
| ALCOHOLS | | | | |
| N-Propanol^{a} | 62 | - | - | - |
| Isopropyl^{a} | - | 70 | - | - |
| Mixtures | | | | |
| (1) N-Propanol^{a} | - | - | 50 | - |
| + Isopropyl^{a} | - | - | 20 | - |
| (2) N-Propanol^{a} | - | - | - | 40 |
| + Ethyl^{a} | - | - | - | 30 |

| EMULSIFIER | | | | |
|---|---|---|---|---|
| Polawax A31^{b} | 4 | - | 4 | - |
| Arlacel 165^{b} | - | 4 | - | 4 |

| DETERGENT | | | | |
|---|---|---|---|---|
| Dodecylammonium chloride^{b},^{c} | 25 | - | - | - |
| Amine Oxide^{b} | - | 10 | - | - |
| Cetylpyridium chloride^{b} | - | - | 10 | - |
| Hyamine 1622^{b} | - | - | - | 10 |

| Mixtures | | | | |
|---|---|---|---|---|
| Laurylamine oxide^{b} | - | - | 5 | 5 |
| Dodecylammonium chloride^{b} | - | 10 | 5 | 5 |

| THICKENING AGENT | | | | |
|---|---|---|---|---|
| Klucel^{b} HFNF (1500-3000) | 1 | 1 | 1 | 1 |

| SCENTS | | | | |
|---|---|---|---|---|
| Phenylethyl alcohol^{b} | 0.25 | 0.25 | 0.25 | 0.25 |
| Alpine^{b} | 0.25 | - | - | - |
| Other^{b} | - | 0.25 | 0.25 | 0.25 |

| PRESERVATIVE | | | | |
|---|---|---|---|---|
| Zinc Omadine^{b},^{d} | 2 | 1 | 2 | 1 |
| Liquipar oil^{b} | 1 | 1 | 1 | 1 |

| ANTIOXIDANT | | | | |
|---|---|---|---|---|
| Tenox Pg^{b} | 0.10 | - | 0.10 | - |
| Tenox S1^{b} | - | 0.10 | - | 0.10 |

| | | | | |
|---|---|---|---|---|
| ^{a} Concentration in percent (v/v). | | | | |
| ^{b} Concentration in percent (w/v). | | | | |
| ^{c} Hydro-alcoholic concentrate. | | | | |
| ^{d} 48% suspension. | | | | |

### EXAMPLE 6: FORMULATION OF PREOPERATIVE ANTISEPTIC AND EVALUATION OF TREATMENT

Five formulations of preoperative antiseptics were prepared according to the procedure given in Example 2 by combining the ingredients given below in Table IX, adjusting the pH to 3.00-4.00 with phosphoric acid, and q.s. to 100 ml with deionized water. The final concentrations are indicated either as percent (v/v) or percent (w/v).

The antimicrobial action of the antiseptic Formulation #5 was compared to that of a soap and water scrub, and an Iodophor (Betadine) scrub, using the FDA patient preoperative skin preparation protocol which specifies the abdomen and groin crease as the test sites. *Federal Register* 59:31450-31452 (June 17, 1994). One contralateral side of the abdomen or groin crease

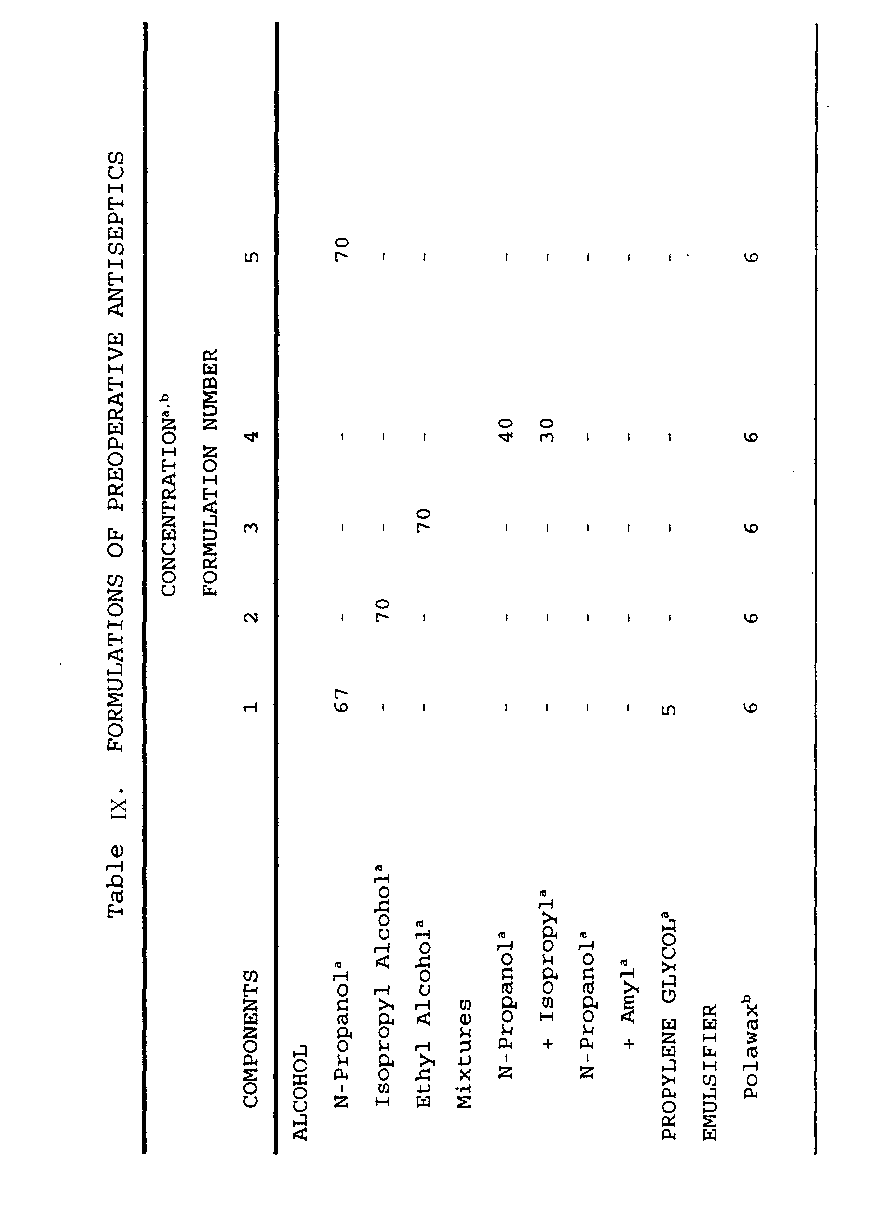

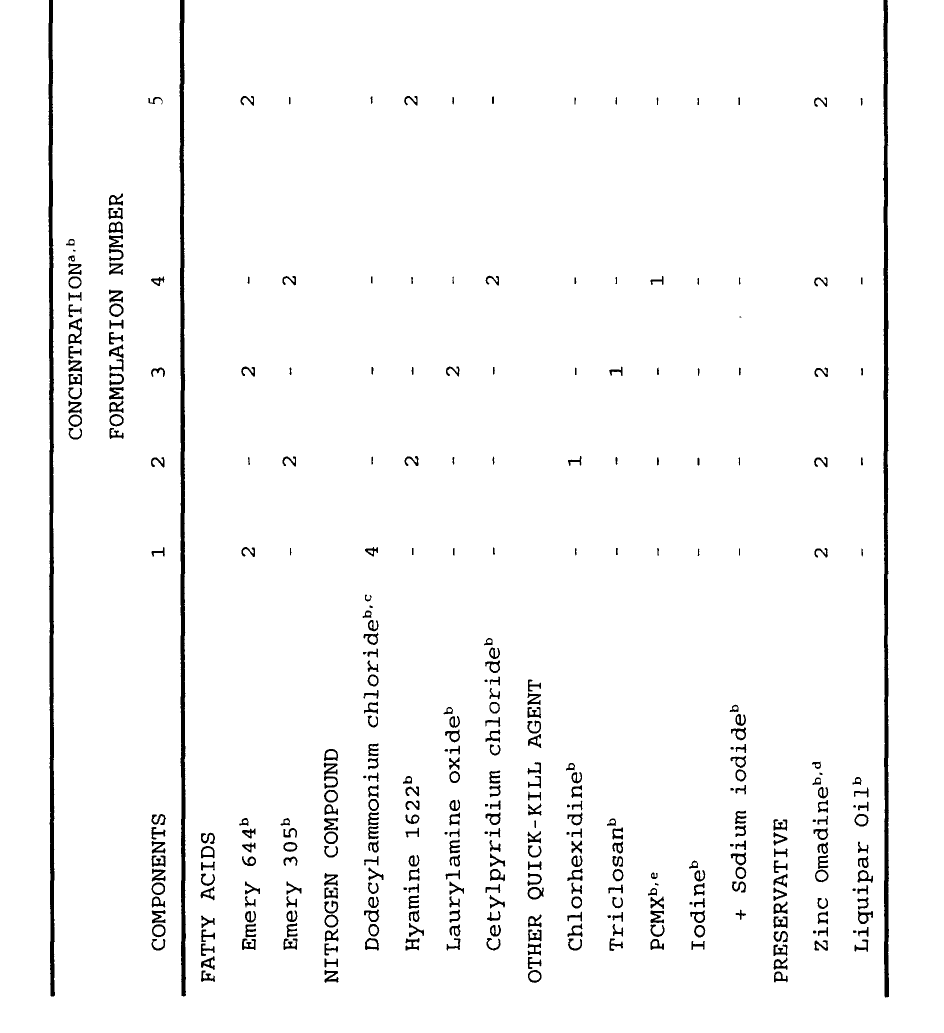

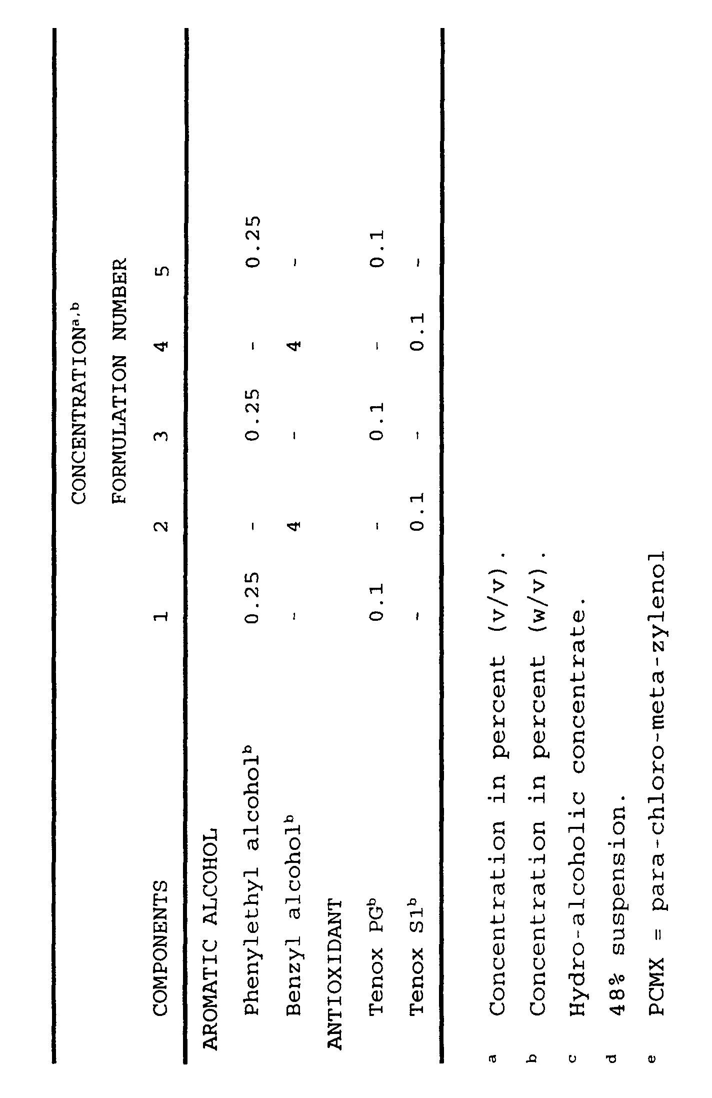

is used as the test site and the other side was used as the baseline control site. Samples were taken at baseline and then at 10 minutes, 6 hours, and 24 hours posttreatment, using the scrubbing cup technique described in Example 6. The scrub time for the antiseptic Scrub formulation and soap and water was three minutes, and the Iodophor was five minutes.

The results from the comparative study (Table X-XV) show that at all time points, the antimicrobial action of the antiseptic was higher than with soap and water, and with iodophor. At ten minutes posttreatment with the antiseptic, there was 59-100% drop in organism count for the abdomen and 87-100% drop for the groin crease, demonstrating very effective quick-kill. Overall, 48-100% drop in organism counts were maintained through twenty-four hours posttreatment with the antiseptic.

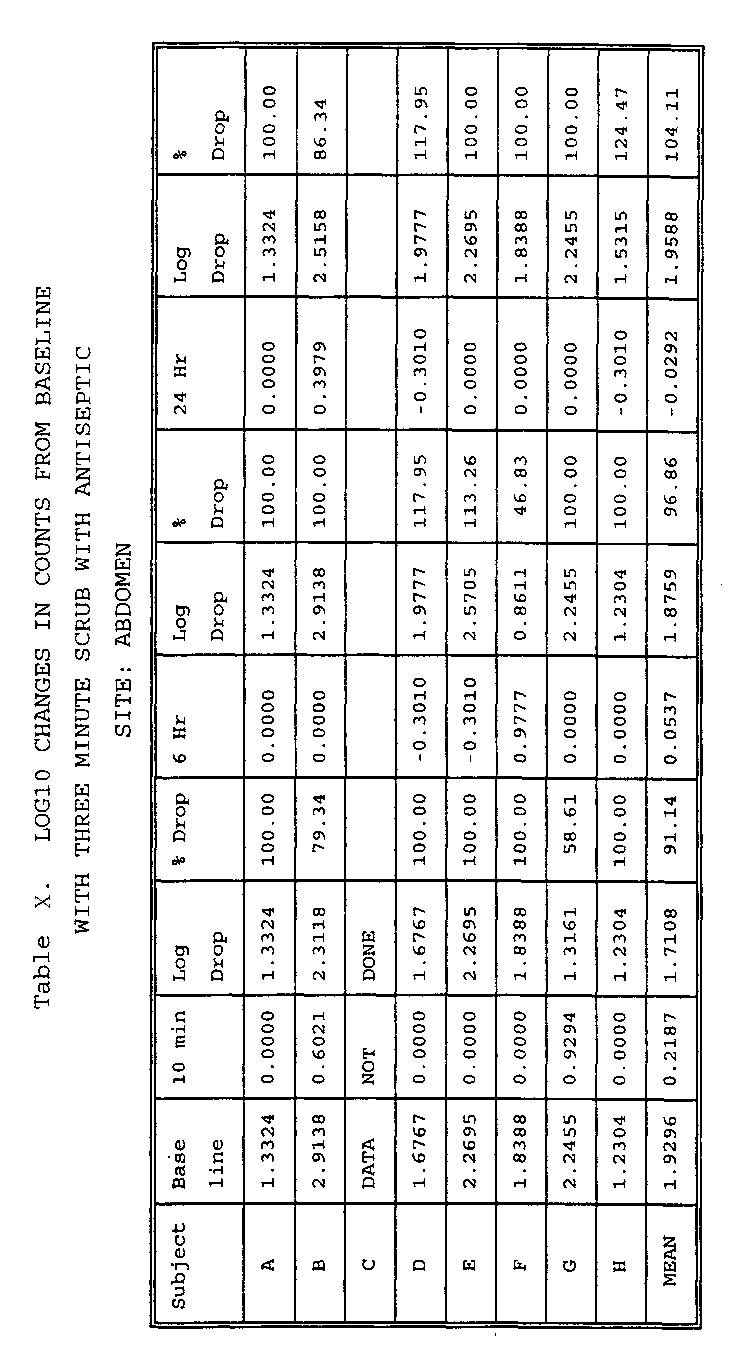

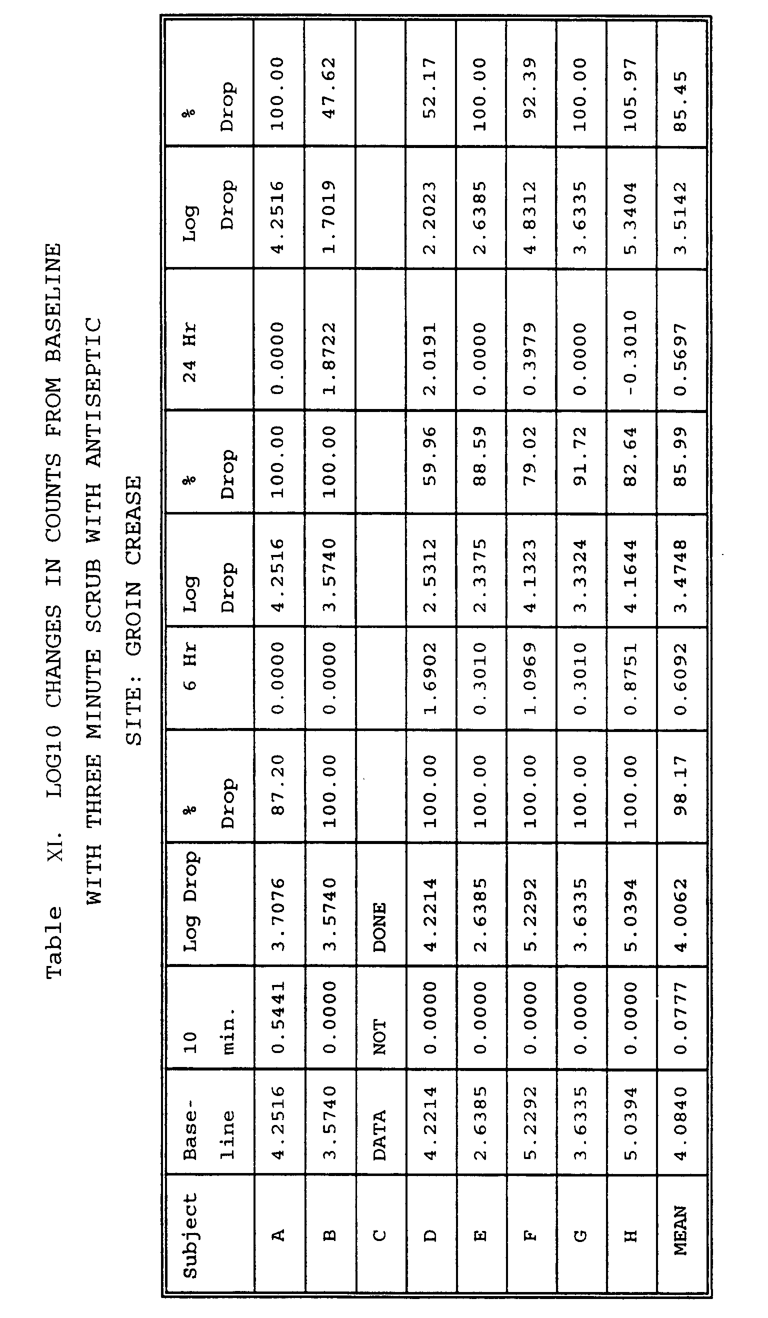

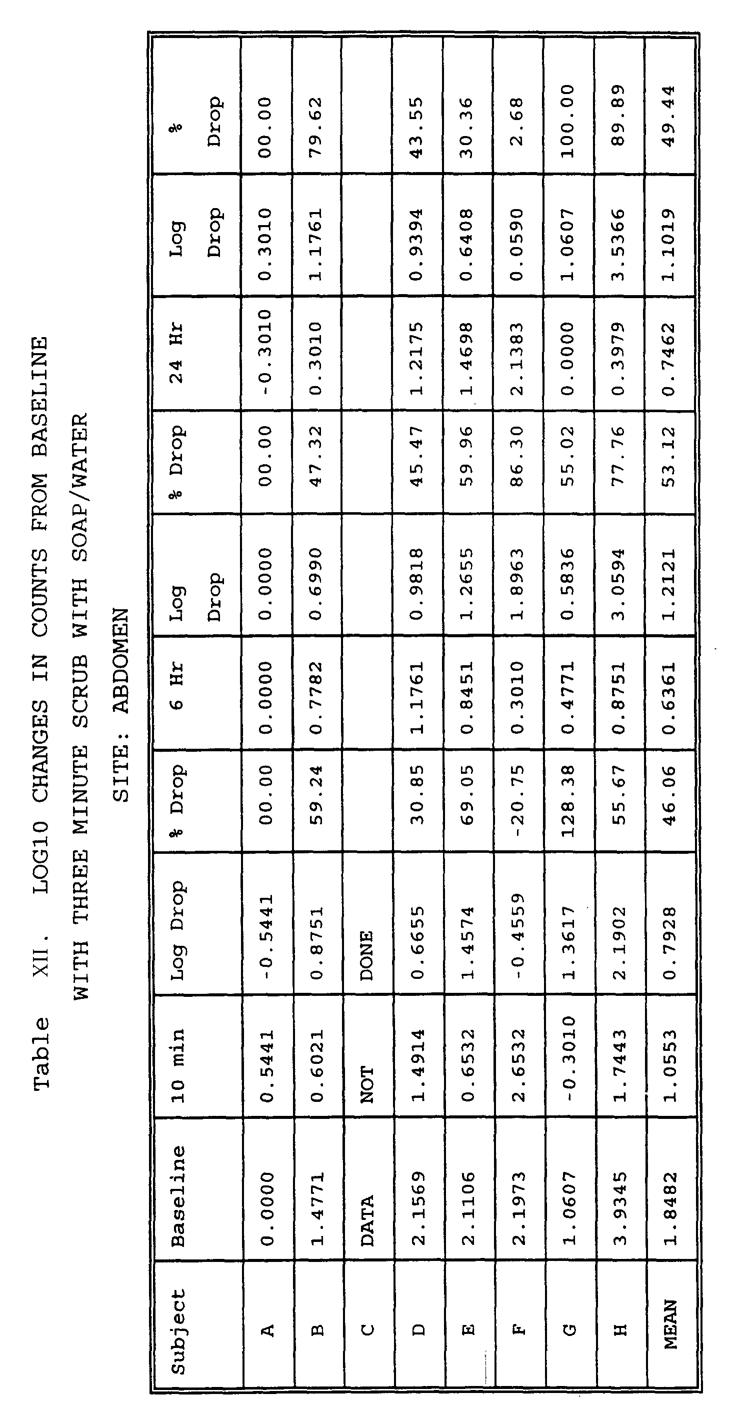

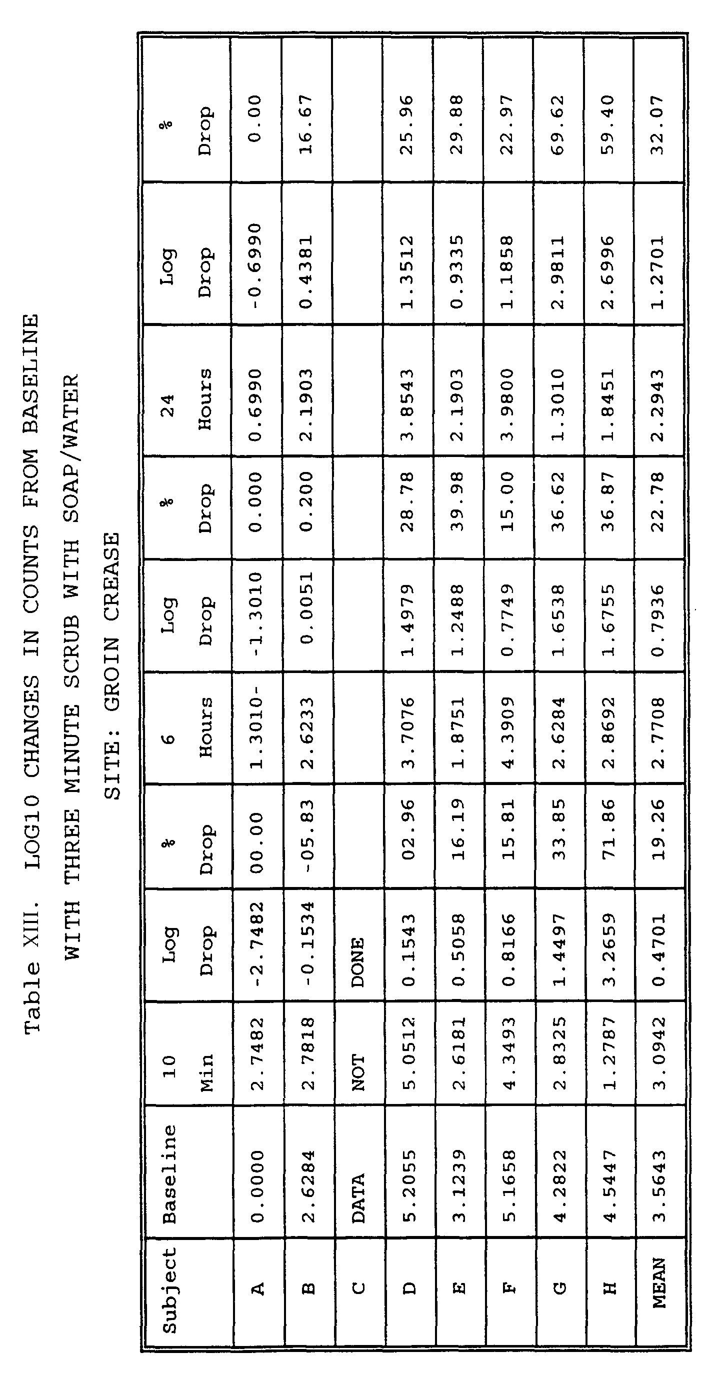

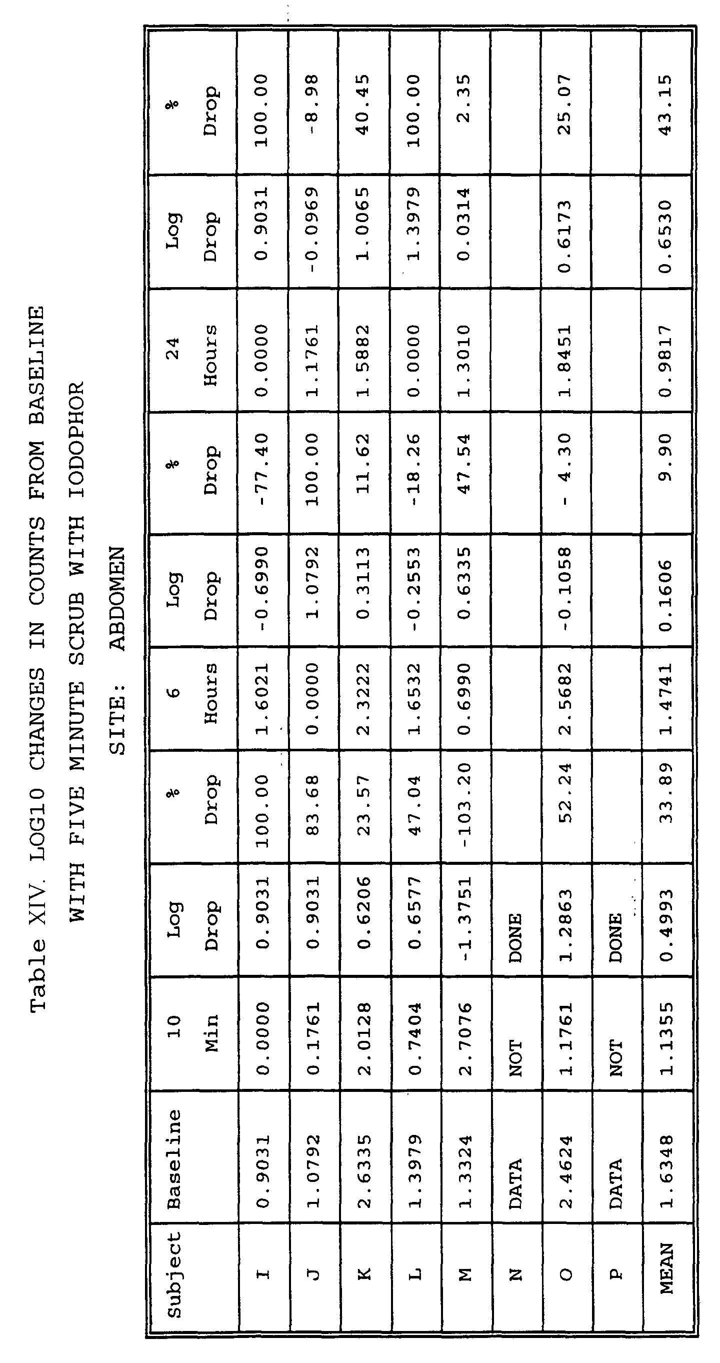

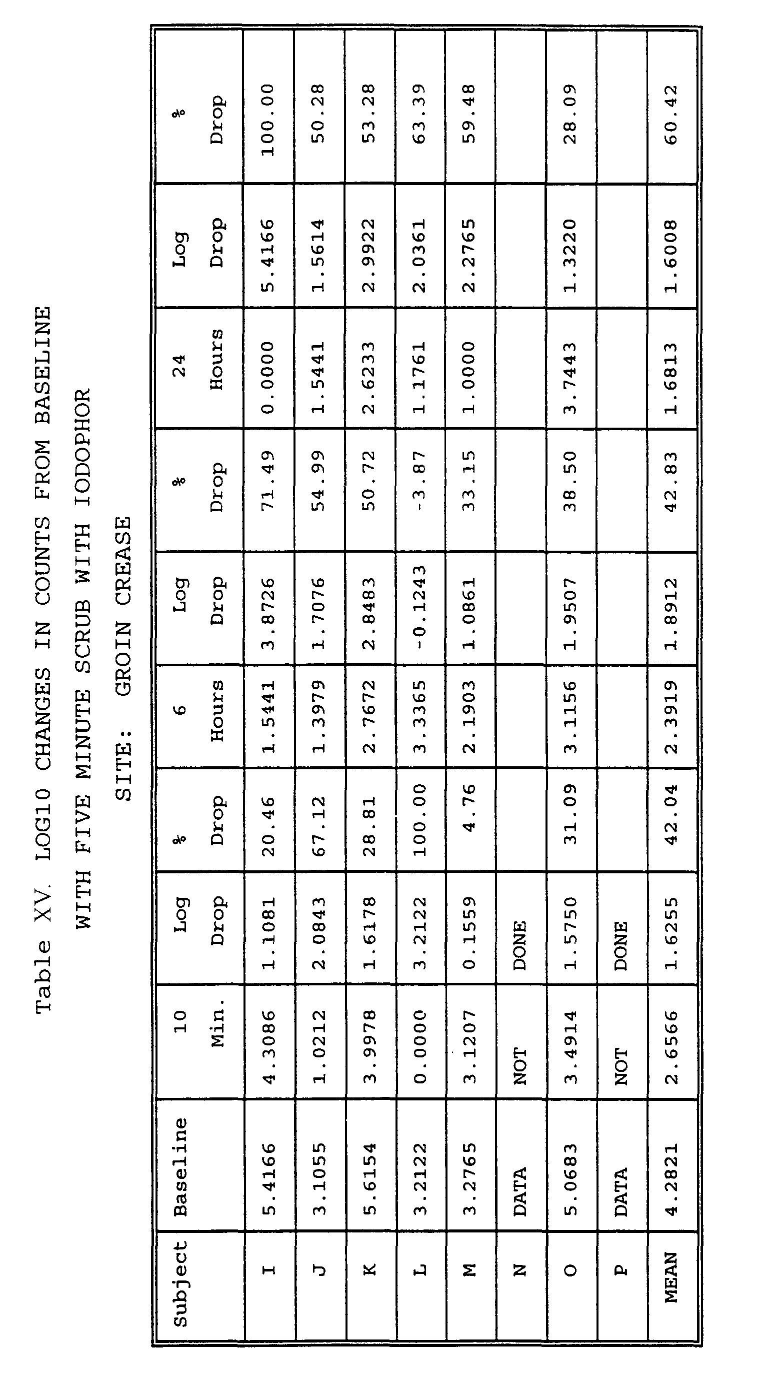

## Claims

1. An antiseptic formulation for topical application, comprising an antimicrobial alcohol selected from the group consisting of ethanol, isopropanol, n-propanol and mixtures thereof; an antimicrobial lipid selected from a group consisting of free fatty acids and fatty acid esters and mixtures thereof; and zinc pyrithione, wherein said antiseptic formulation has an acidic pH.

2. The antiseptic formulation of claim 1, wherein said antimicrobial alcohol is n-propanol.

3. The antiseptic formulation of claim 1 or 2, wherein said antimicrobial lipid is selected from the group consisting of free fatty acids having 2 to 20 carbons and fatty acid esters having 2 to 24 carbons and mixtures thereof.

4. The antiseptic formulation of claims 1, 2 or 3, further comprising an emulsifying agent selected from the group consisting of ethoxylated cetyl alcohol, ethoxylated stearyl alcohol and mixtures thereof, and emulsifying waxes essentially comprising cetyl and stearyl alcohols.

5. An antiseptic formulation for topical application, comprising an antimicrobial alcohol selected from the group consisting of ethanol, isopropanol, n-propanol and mixtures thereof; a bispyrithione compound; and an emulsifying agent selected from the group consisting of ethoxylated cetyl alcohol, ethoxylated stearyl alcohol and mixtures thereof, and emulsifying waxes essentially comprising cetyl and stearyl alcohols.

6. The antiseptic formulation of claim 5, wherein said bispyrithione compound is selected from the group consisting of zinc pyrithione, magnesium pyrithione and mixtures thereof.

7. The antiseptic formulation of claims 5 or 6, wherein said formulation has an acidic pH.

## Patentansprüche

1. Antiseptische Formulierung zur topischen Applikation, umfassend einen antimikrobiellen Alkohol, der aus der Gruppe bestehend aus Ethanol, Isopropanol, n-Propanol und Mischungen davon ausgewählt ist; ein antimikrobielles Lipid, das aus der Gruppe bestehend aus freien Fettsäuren und Fettsäureestern und Mischungen davon ausgewählt ist; und Zinkpyrithion, wobei die antiseptische Formulierung einen sauren pH-Wert hat.

2. Antiseptische Formulierung nach Anspruch 1, wobei der antimikrobielle Alkohol n-Propanol ist.

3. Antiseptische Formulierung nach Anspruch 1 oder 2, wobei das antimikrobielle Lipid aus der Gruppe bestehend aus freien Fettsäuren mit 2 bis 20 Kohlenstoffen und Fettsäureestern mit 2 bis 24 Kohlenstoffen und Mischungen davon ausgewählt ist.

4. Antiseptische Formulierung nach den Ansprüchen 1, 2 oder 3, ferner umfassen ein Emulgierungsmittel, ausgewählt aus der Gruppe bestehend aus ethoxyliertem Cetylalkohol, ethoxyliertem Stearylalkohol und Mischungen davon, und emulgierende Wachse, die im wesentlichen Cetyl- und Stearylalkohole umfassen.

5. Antiseptische Formulierung zur topischen Applikation, umfassend einen antimikrobiellen Alkohol, ausgewählt aus der Gruppe bestehend aus Ethanol, Isopropanol, n-Propanol und Mischungen davon; eine Bispyrithion-Verbindung; und ein Emulgierungsmittel, ausgewählt aus der Gruppe bestehend aus ethoxyliertem Cetylalkohol, ethoxyliertem Stearylalkohol und Mischungen davon, und emulgierende Wachse, die im wesentlichen Cetyl- und Stearylalkohole umfassen.

6. Antiseptische Formulierung nach Anspruch 5, wobei die Bispyrithion-Verbindung aus der Gruppe bestehend aus Zinkpyrithion, Magnesiumpyrithion und Mischungen davon ausgewählt ist.

7. Antiseptische Formulierung nach den Ansprüchen 5 oder 6, wobei die Formulierung einen sauren pH-Wert hat.

## Revendications

1. Formulation antiseptique pour application locale, comprenant un alcool antimicrobien choisi dans le groupe constitué par l'éthanol, l'isopropanol, le n-propanol et les mélanges de ceux-ci ; un lipide antimicrobien choisi dans un groupe constitué par des acides gras libres et des esters d'acide gras et les mélanges de ceux-ci ; et de la pyrithione de zinc, laquelle formulation antiseptique a un pH acide.

2. Formulation antiseptique selon la revendication 1, dans laquelle ledit alcool antimicrobien est le n-propanol.

3. Formulation antiseptique selon la revendication 1 ou 2, dans laquelle ledit lipide antimicrobien est choisi dans le groupe constitué par les acides gras libres ayant de 2 à 20 carbones et les esters d'acide gras ayant de 2 à 24 carbones et les mélanges de ceux-ci.

4. Formulation antiseptique selon la revendication 1, 2 ou 3, comprenant en outre un agent émulsionnant choisi dans le groupe constitué par l'alcool cétylique éthoxylé, l'acool stéarylique éthoxylé et les mélanges de ceux-ci, et des cires émulsionnantes comprenant essentiellement des alcools cétylique et stéarylique.

5. Formulation antiseptique pour application locale, comprenant un alcool antimicrobien choisi dans le groupe constitué par l'éthanol, l'isopropanol, le n-propanol et les mélanges de ceux-ci ; un composé de bispyrithione ; et un agent émulsionnant choisi dans le groupe constitué par l'alcool cétylique éthoxylé, l'acool stéarylique éthoxylé et les mélanges de ceux-ci, et des cires émulsionnantes comprenant essentiellement des alcools cétylique et stéarylique.

6. Formulation antiseptique selon la revendication 5, dans laquelle ledit composé de bispyrithione est choisi dans le groupe constitué par la pyrithione de zinc, la pyrithione de magnésium et les mélanges de celles-ci.

7. Formulation antiseptique selon les revendications 5 ou 6, laquelle formulation a un pH acide.
